# EUROPEAN PATENT APPLICATION

(11) **EP 2 572 723 A2**
(43) Date of publication of application: **27.03.2013**
(21) Application number: 12179498.6
(22) Date of filing: 20.08.2007
(51) Int. Cl.: A61K 38/46

(54) **Materials and methods for treating or preventing organophosphate exposure associated damage**

(62) Divisional of application: 07805492.1
(71) Applicant: Yissum Research and Development Company of The Hebrew University of Jerusalem, 91390 Jerusalem (IL); Arizona Board Of Regents, A Body Corporate Acting For And On Behalf Of Northern Arizona University, Scottsdale, AZ 85257-8538 (US)
(72) Inventor: Soreq, Hermona, 95903 Jerusalem (IL); Mor, Tsafrir, Tempe, AZ Arizona 85282 (US)
(74) Representative: Dennemeyer & Associates S.A.

(57) **Abstract**

The present invention relates to the use of AChE-R for the treating or preventing organophosphate exposure associated damage, wherein said AChE-R is (a) recombinant or (b) plant produced or both; and (c) as set forth in SEQ ID NO: 4.

## Description

### FIELD AND BACKGROUND OF THE INVENTION

The present invention relates to materials and methods for treating or preventing organophosphate exposure associated damage.

Accidental (environmental or occupational) and self-inflicted (suicide) exposure to organophosphate (OP) pesticides is encountered frequently in the emergency room, especially in the developing world. These perennial public health issues are compounded by a growing concern over the potential use of OP nerve agents such as sarin as a means of terror and nonconventional warfare. OPs disrupt neurotransmission by inhibiting synaptic acetylcholinesterase (AChE-S), leading to the accumulation of acetylcholine in the synapse and neural over-stimulation. The severity of the ensuing nicotinic and muscarinic symptoms is dose-dependent and can result in death due to cardiovascular and respiratory collapse. Those surviving the initial insult often suffer long-term sequelae, including OP-induced delayed neuropathy, muscle weakness, permanent brain dismorphology and social/behavioral deficits.

The mechanism underlying OP-delayed toxicity and other stressful insults (e.g. psychological stress or head trauma), involve rapid elevation of *c-fos* followed by up-regulation of the *ACHE* gene [Friedman, 1996, Nat. Med. 2,1382-1385].

Human and mouse AChE pre-mRNA harbors alternative splicing sites at both 3' and 5' ends, the latter with corresponding promoters. Alternative splicing at the 3' ends yields three AChE variants with different carboxy terminal sequences which determine the protein product multimerization capacities and its cellular localization. Synaptic AChE-S, the main transcript expressed in the nervous system and neuromuscular junctions (NMJs) contains a C-terminal cysteine, allowing dimerization by disulphide bonds. This dimer can form tetramers by the attachment of additional monomers and can adhere to the membrane through the CoIQ and PRiMA structural subunits. The erythrocytic, AChE-E transcript includes a glycyl bond in its C-terminus which allows the attachment of a glycophosphatidylinositol group to the protein, followed by its anchoring to erythrocyte membrane as a dimer. The normally rare, stress-induced "readthrough", AChE-R variant remains a soluble monomer (see e.g., U.S. Pat. No. 5,932,780).

Regulation of AChE is primarily at the transcriptional level, although some post-transcriptional regulation has been reported, with the latter being generally associated with attempted compensation to neurodegenerative disease. The AChE locus contains several putative upstream enhancers, including those belonging to NF-κB, AP-1 and a glucocorticoid response element. Conditions that increase transcription include cellular differentiation and anticholinesterase exposure. Regulation of isoform switching is much more complex and less well understood. In mammalian physiology, AChE-S is the the dominant transcript and AChE-R is extremely rare. In addition to increasing AChE transcription globally, physical, chemical and psychological stress increase the proportion of AChE-R relative to AChE-S.

It has been shown that following OP-delayed toxicity there is a rapid, yet long-lasting shifted alternative splicing from AChE-S to the otherwise rare "readthrough" variant (AChE-R) [Kaufer, D., et al., 1998, Nature 393, 373-377; Shohami, E., 2000, J Mol Med 78, 228-236; Meshorer, E., 2002, Science 295, 508-512]. Johnson et al.. {Environmental Health Perspectives, 115, 1, 2007], teach up-regulation of both AChE-S and AChE-R following exposure to non-symptomatic doses of OPs. Perrier et al., [J of Neurochemistry. 2005, 94, 629-638] teach a moderate increase in AChE-R following OP exposure, without observing changes in the activity of the enzyme

However, until presently, it was unknown whether the post-exposure changes in AChE gene expression (i.e. the overall increase and the alternative splicing shift from AChE-S to AChE-R), reflect exposure-induced damages or, inversely, adaptive reactions with protective value.

Existing treatment protocols emphasize three complementary approaches: Antagonize the muscarinic ACh receptor with atropine, reactivate endogenous AChE with oxime therapy, and manage the severe toxicity symptomatically. While these approaches have proven value in reducing mortality, they are unable to prevent the debilitating morbidity or long-term consequences that are associated with anticholinesterase poisoning.

As an alternative, the use of human cholinesterases (ChEs) as OP bioscavengers has been suggested [Maxwell, 1993, J. Pharmacol. Exp. Ther. 264, 1085-1089], but the practicality of this approach depends on the availability of large amounts of these enzymes that are required in stoichiometric, rather than catalytic, quantities. The considerable success with plasma-purified butyrylcholinesterase (BChE) as an OP bioscavenger [Ashani, Y., 2000, Drug Dev. Res. 50, 298-308; Doctor, B. P., and Saxena, A., 2005, Chem Biol Interact In press], called for comparative studies with the physiologically relevant target of OPs, human AChE. AChE shows considerably faster OP-binding kinetics than BChE [Maxwell, 1993, J. Pharmacol. Exp. Ther. 264, 1085-1089], predicting that smaller doses may be effective [Kaplan et al., Biochemistry 40, 7433-7445].

### SUMMARY OF THE INVENTION

According to one aspect, there is provided a method of treating or preventing organophosphate exposure associated damage in a subject in need thereof, the method comprising providing the subject with a therapeutically effective amount of AChE-R to thereby treat the organophosphate exposure associated damage in the subject.

According to another aspect, there is provided an article of manufacture for treating or preventing organophosphate exposure associated damage, the article of manufacture comprising AChE-R immobilized on to a solid support.

According to yet another aspect, there is provided a method of detoxifying a surface, the method comprising contacting the surface with AChE-R, thereby detoxifying the surface.

According to still another aspect, there is prov/ided a coating material comprising AChE-R.

According to one embodiment, the AChE-R comprises ARP.

According to another embodiment, the AChE-R comprises recombinant AChE-R.

According to another embodiment, the recombinant AChE-R is plant produced AChE-R.

According to another embodiment, the AChE-R is as set forth in SEQ ID NOs. 2 and 4.

According to another embodiment, the providing comprises administering to the subject the AChE-R.

According to another embodiment, the providing is effected prior to the organophosphate exposure.

According to another embodiment, the administering is effected by inhalation.

According to another embodiment, the administering is effected 10 hours prior to the exposure until 7 days following exposure.

According to another embodiment, the administering is effected by inhalation and injection.

According to another embodiment, the method further comprises administering to the subject atropine and optionally oxime.

According to another embodiment, the providing is effected by topical application.

According to another embodiment, the solid support is for topical administration.

According to another embodiment, the solid support for topical administration is selected from the group consisting of a sponge, a wipe and a fabric

According to another embodiment, the solid support is selected from the group consisting of a filter, a fabric and a lining.

According to another embodiment, the method further comprises contacting the surface with a caustic agent; a decontaminating foam, a combination of baking condition heat and carbon dioxide, or a combination thereof

According to another embodiment, the AChE-R is comprised in a coating, a paint, a non-film forming coating, an elastomer, an adhesive, an sealant, a material applied to a textile, or a wax.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

As used herein, the terms "comprising"' and "including" or grammatical variants thereof are to be taken as specifying the stated features, integers, steps or components but do not preclude the addition of one or more additional features, integers, steps, components or groups thereof. This term encompasses the terms "consisting of' and "consisting essentially of'.

The phrase "consisting essentially of' or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than, is necessary for a fundamental understanding of the invention, the description taken with the drawings making apparent to those skilled in the art how the several forms of the invention may be embodied in practice.

In the drawings:

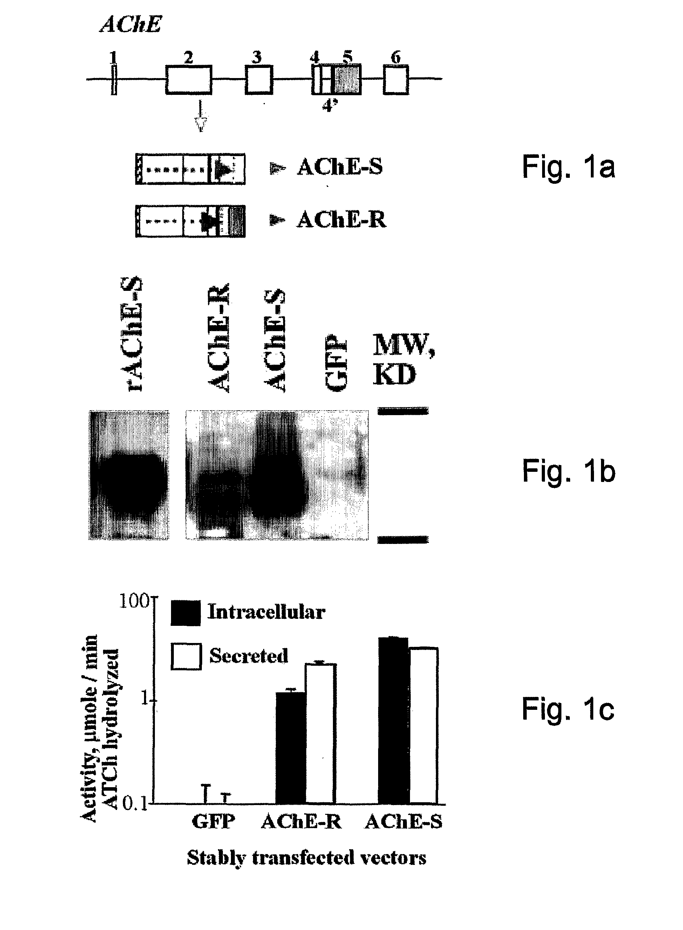

FIGS. 1A-C illustrate the establishment of stably transfected cell lines overexpressing human AChE variants. Figure 1A is a scheme showing the alternatively spliced variants of the AChE gene. Arrows show the protein product of the respective mRNAs. Figure 1B is a Western blot of total extracts of transfected CHO cells probed with an antibody (N-19, Santa Cruz Biotechnology, Santa Cruz, CA) against the common region of the AChE protein. rAChE-S, commercially purified recombinant AChE-S (Sigma-Aldrich, Rehovot, Israel) served as control. Figure 1C is a bar graph illustrating total AChE activity in CHO cells stably transfected with constructs carrying either the AChE-R or AChE-S cDNA under the control of the minimal CMV enhancer-promoter. CHO- cells stably transfected with a GFP vector (GFP) served for control. Solid bars show intracellular activity in µM hydrolyzed ACh/ mg protein. Empty bars show extracellular activity in µM hydrolyzed ACh/ ml medium. Error bars show Standard Deviation.

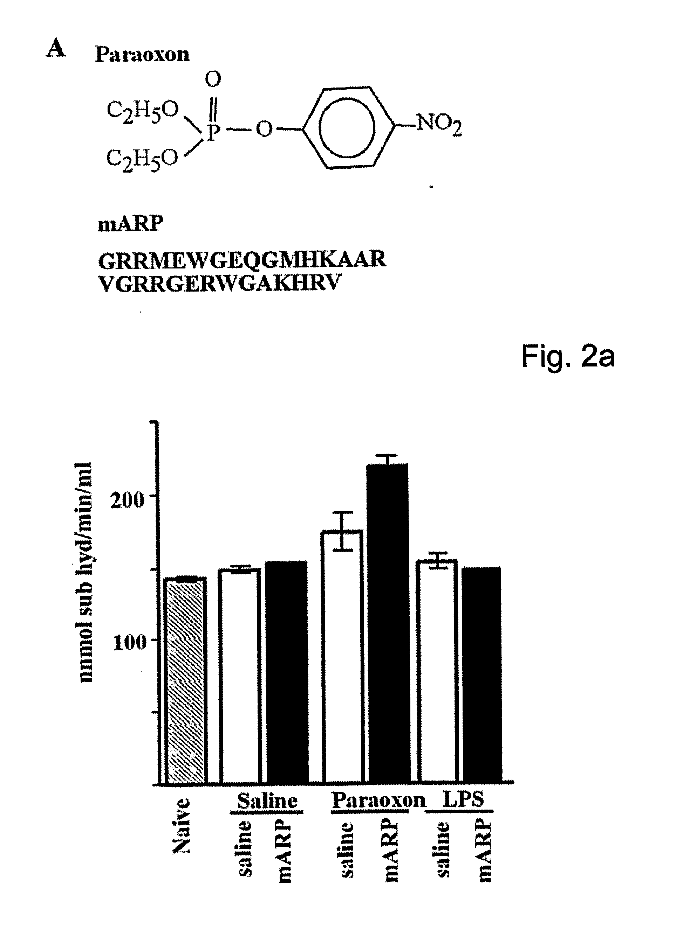

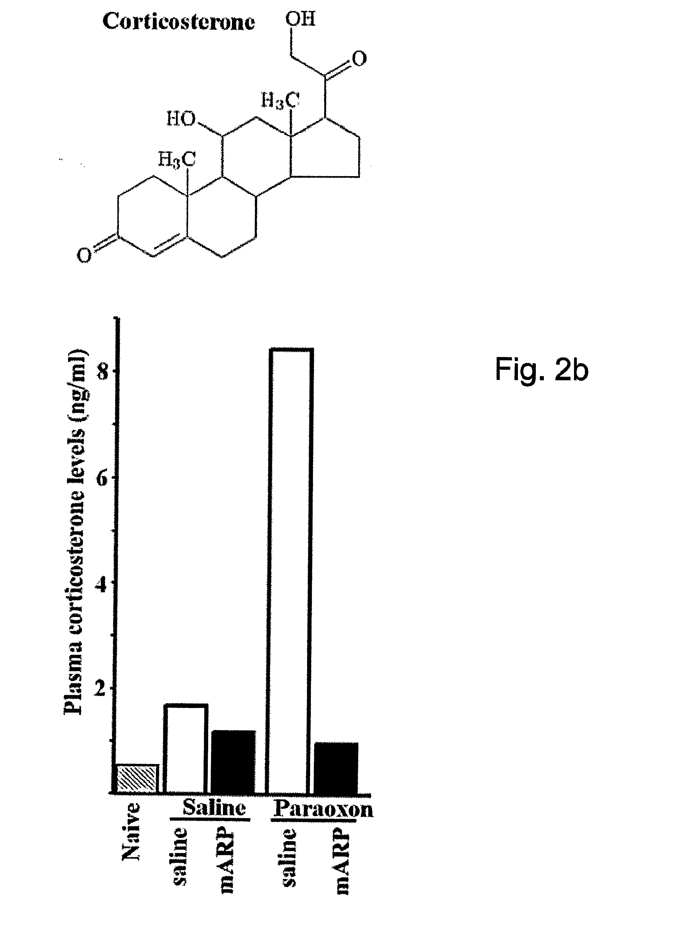

FIGs. 2A-B are bar graphs illustrating plasma AChE activities and corticosterone levels post-exposure. Figure 2A: AChE specific activities were measured in plasma of all animals at day 7 post-treatment using Ellman's assay and in the presence of 50 µM ISO-OMPA. The largest enhancement in plasma AChE activities was observed following co-treatment with Paraoxon and mARP. Insets: Paraoxon structure and the mARP sequence. Figure 2B: Plasma corticosterone levels were measured in plasma at day 6 of the experiment using the OCTEIA Corticosterone kit (IDS Ltd, Boldon, UK) according to the manufacturer's instructions. mARP ameliorated the increase in corticosterone levels that was induced by saline and paraoxon. Inset: Corticosterone structure.

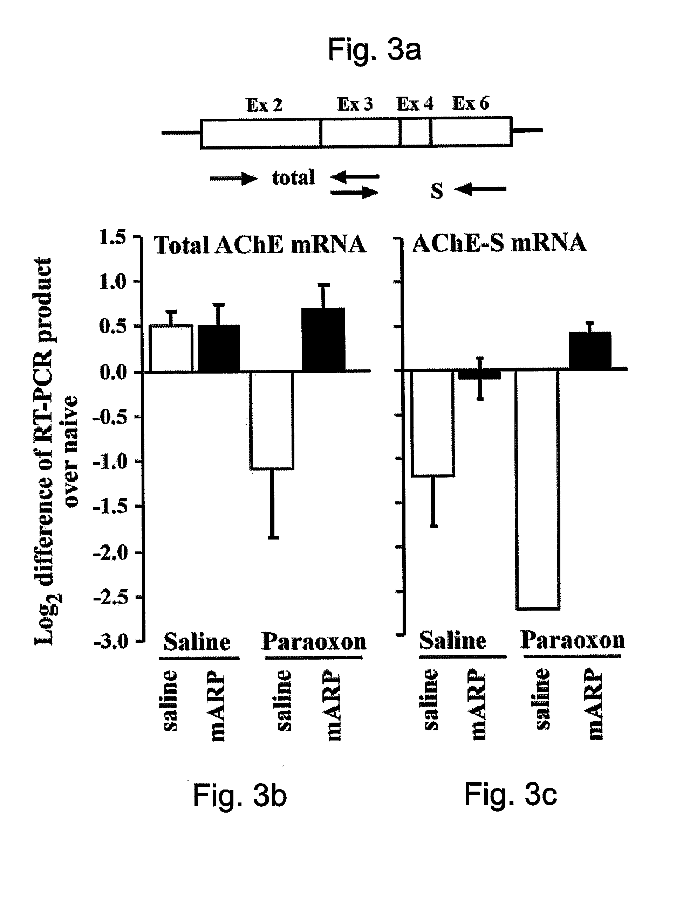

FIGs. 3A-C illustrates that mARP ameliorates exposure effects on AChE gene expression. AChE mRNA levels were measured in skeletal muscles of treated animals at the end of one week of experiment. Total RNA was purified from frozen skeletal muscles and subjected to real-time RT-PCR using the Light Cycler system (Roche Diagnostics, Mannheim, Germany) for real-time PCR quantification. Figure 3A iilustrates the positions of the two sets of primers used to amplify both the commom domain of AChE mRNA (Figure 3B) and the 3' region that is unique for AChE-S mRNA (Figure 3C). Numbers denote nucleotide positions. Columns present the log₂ differences of RT-PCR product quantities over naïve animals, measured as cycle numbers. Values were calibrated to those of the standard transcript, β-actin mRNA. Bar graphs thus present logarithmic differences in product accumulation as compared to a baseline in naïve mice. A positive value reflects an increase in the mRNA levels within the tested muscle samples. Each bar presents an average of 3 animals with the exception of the bar of Paraoxon + saline in the right panel, where only 2 animals were available for this experiment. The third mouse presented muscle AChE-S mRNA levels that were too low to be quantified, emphasizing the observed decrease.

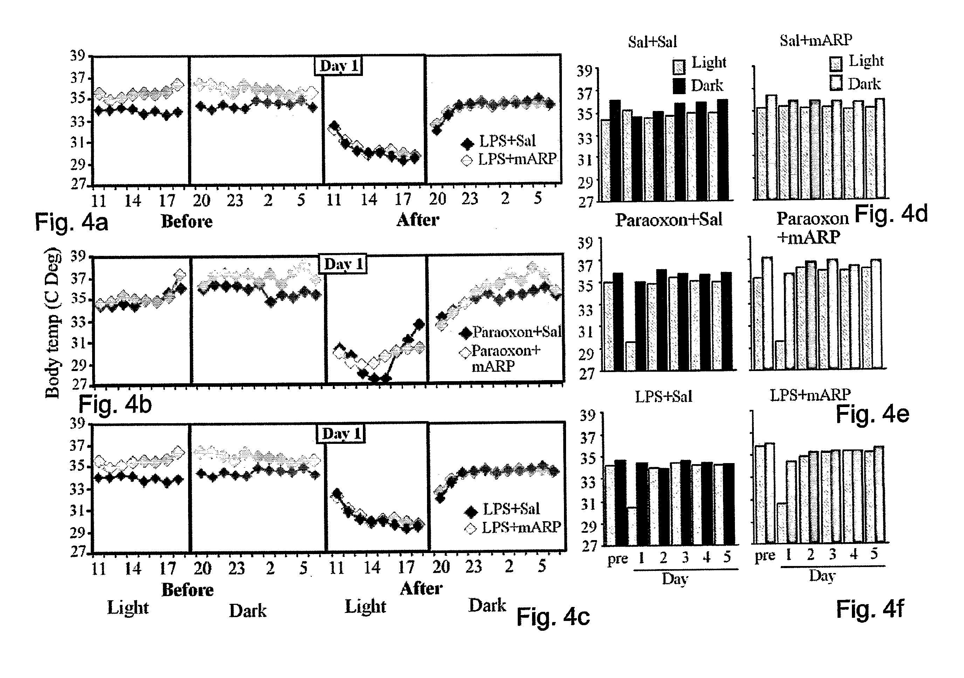

FIGs. 4A-F are graphs illustrating that mARP alters exposure effects on body temperature (BT). At the end of adjustment period, three animals per tested group were subjected to telemetric transmitter implantation followed by 2 weeks recovery period. Following recovery, recording was begun for the entire duration of the experiment. Two days after establishing telemetric baseline values, the mice were injected with saline, 0.4 mg/kg paraoxon, i.p. (Sigma) or 2.5 mg/kg *E. coli* lipopolysaccharide (LPS), *i.p.* Daily throughout the experiment, the mice were injected *i.v;* with mg/kg of mARP or with saline for control. Telemetrically measured BT is shown for the last pre- and first post-treatment days (Figures 4A-C). Note the ameliorated LPS-induced decrease on day 1. Bar graphs show average daily temperatures throughout the entire treatment period (Figures 4D-F).

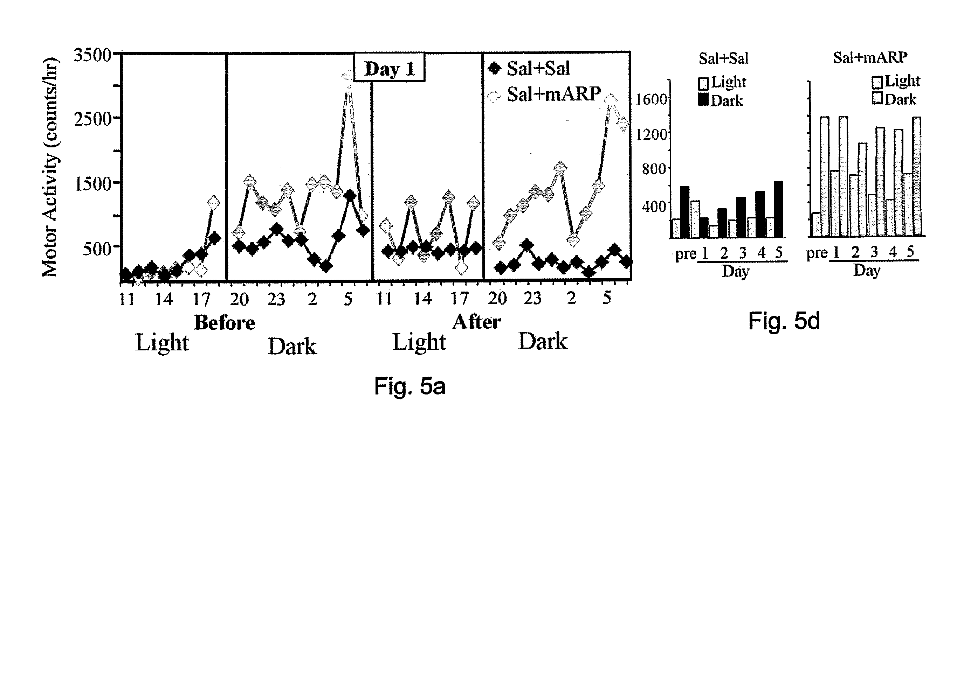

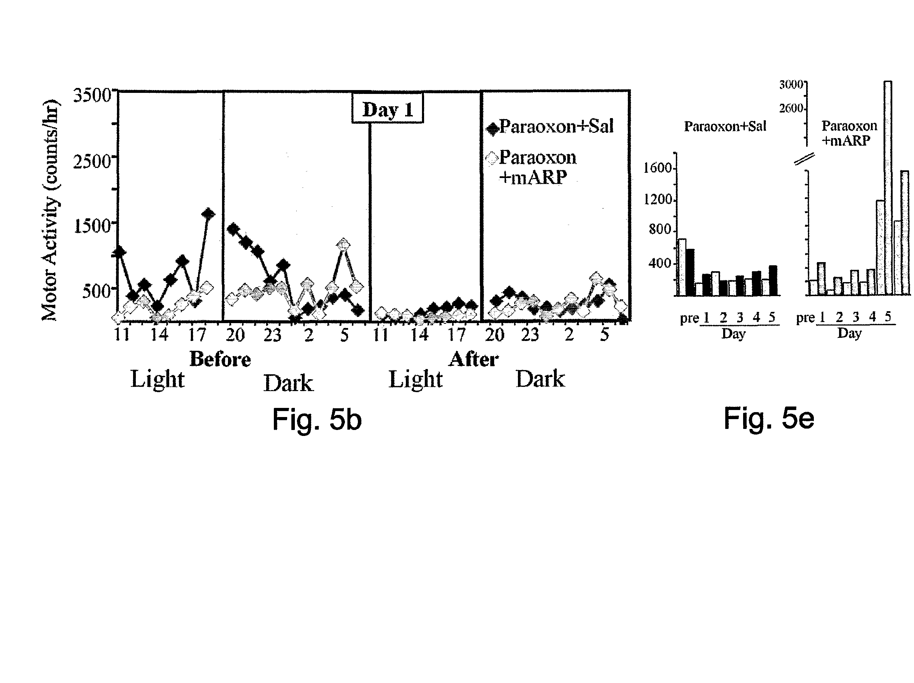

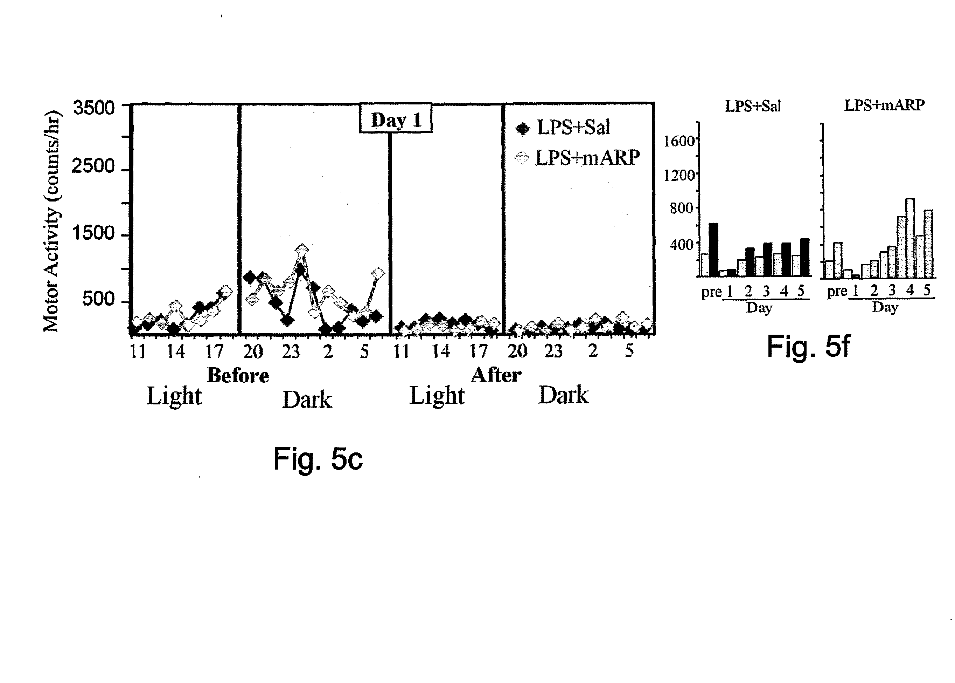

FIGs. 5A-F are graphs illustrating mARP effects on motor activity (MA). Figures 5A-C show MA during the pre-treatment day and the first post-treatment day. Figures 5D-F are bar graphs indicating the avearge light- and dark-phase MA over the course of the complete experiment, both pre- and post-treatment. Shown is the number of events monitored by the implanted transmitter as it moves across a sensing platform under the mouse cage.

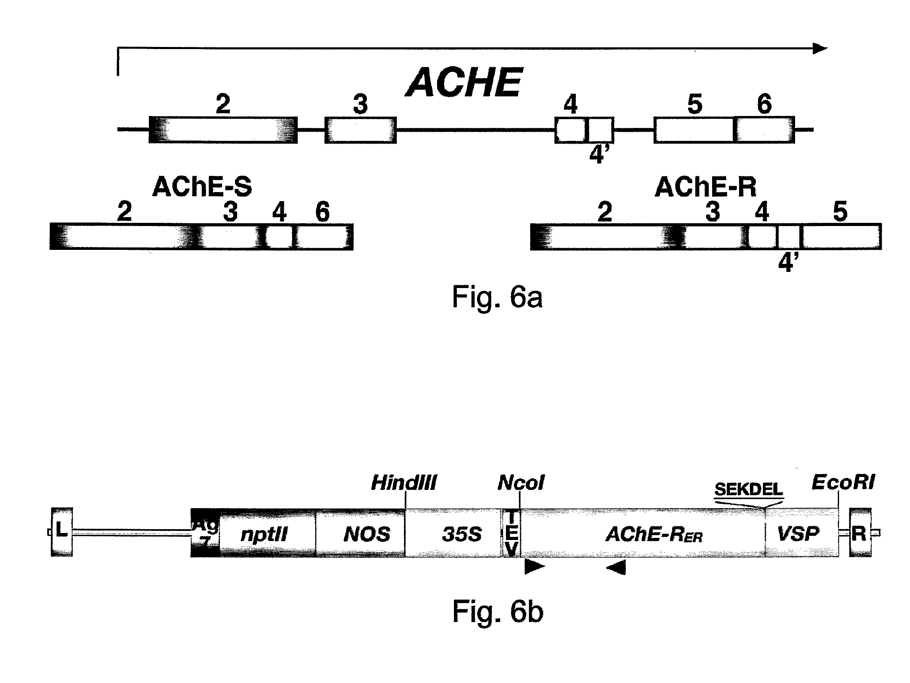

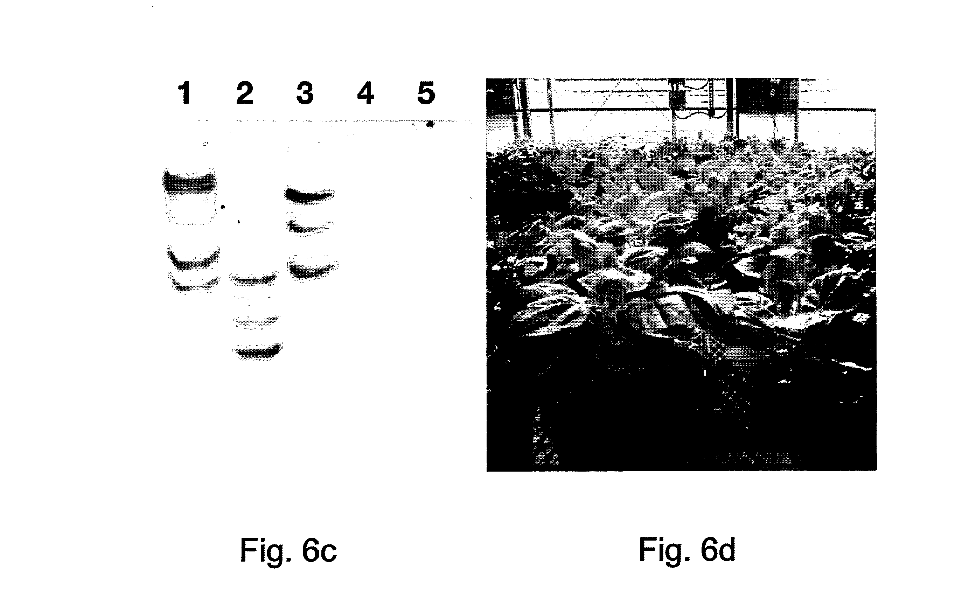

FIGs. 6A-D are diagrams and photographs illustrating the expression of AChE-R_{ER} in plants. Figure 6D is a model illustrating that AChE-S and AChE-R are alternative splicing variants with distinct C-termini. Figure 6B is a diagrams illustrating the AChE-R_{ER} expression vector. L. left border; R, right border; Ag7, the nopline synthase polyadenylation signal; nptII, the kanamycin resistance marker; NOS, the nopline synthase promoter; 35S, cauliflower mosaic virus 35S promoter; TEV, the translation enhancer region of the tobacco etch virus; AChE-R_{ER}, codon-optimized coding region for human AChE-R, containing the C-terminal SEKDEL endoplasmic reticulum retention signal; VSP, the 3' UTR of the soybean vegetative storage protein; arrowheads, locations of primers used to generate probe for DNA blot analysis. Figure 6C is a DNA blot analysis. Genomic DNA was extracted from the highest expressing *N. benthamiana* line (2D) (lanes 1-3) and wt plants (lanes 4-5) and digested with *Eco*RI (lanes 1 & 4), *Nco*I (lanes 2 &5) and *Hind*III (lane 3). Figure 6D is a photograph illustrating large-scale cultivation of 2D *N. benthamiana* in a controlled growth facility at Arizona State University.

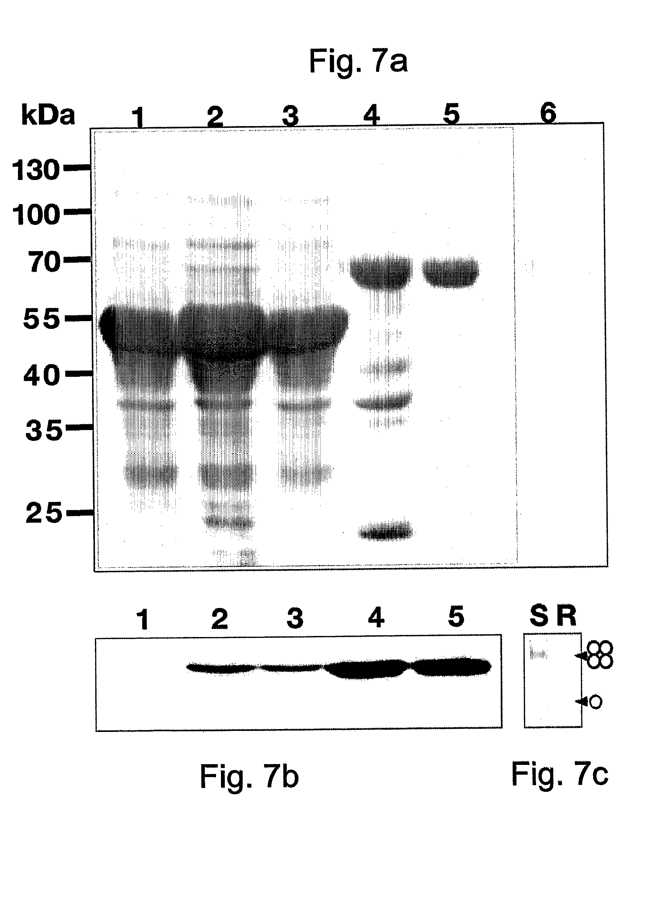

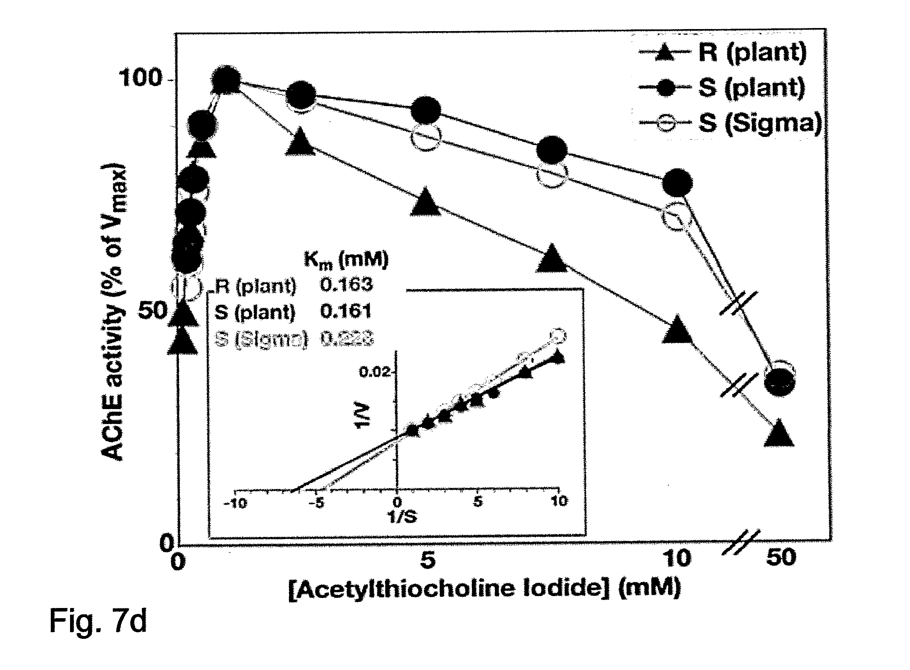

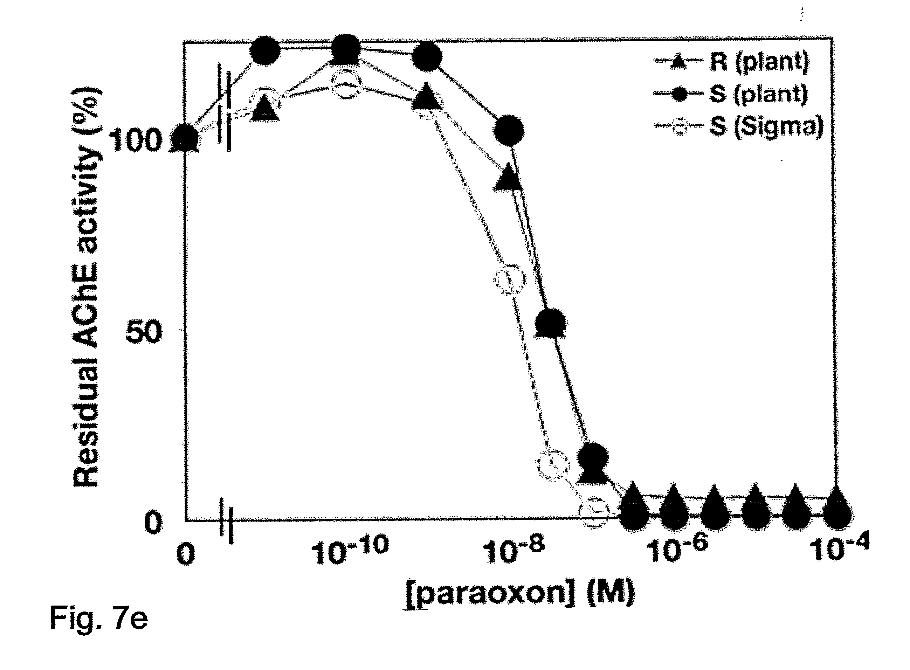

FIGs. 7A-E are photographs and graphs illustrating the characterization of plant-produced AChE-R_{ER}. Figure 7A: Fractions from successive purification steps were subjected to SDS-PAGE and visualized by Coomassie staining (lanes 1-5) or silver staining (lane 6) as follows: Crude wt extract (lane 1), crude 2D extract (lane 2), procainamide affinity chromatography flowthrough (lane 3), eluate following extensive dialysis (lane 4), final product following anion-exchange chromatography and concentrating (lanes 5 and 6). Figure 7B: The above fractions were subjected to immunoblot analysis (lanes 1-5) with AChE-specific Abs. Figure 7C: non-denaturing PAGE stained for ChE activity. Figure 7D: Plant-produced AChE-R_{ER} displays equivalent K*ₘ* and substrate inhibition as compared to mammalian cell-culture or plant produced AChE-S. Insert: Lineweaver-Burke analysis (insert). Figure 7E: Residual activities of plant-derived AChE-R_{ER} and AChE-S and mammalian cell-culture derived AChE-S were assayed in the presence of the indicated concentration of the OP paraoxon.

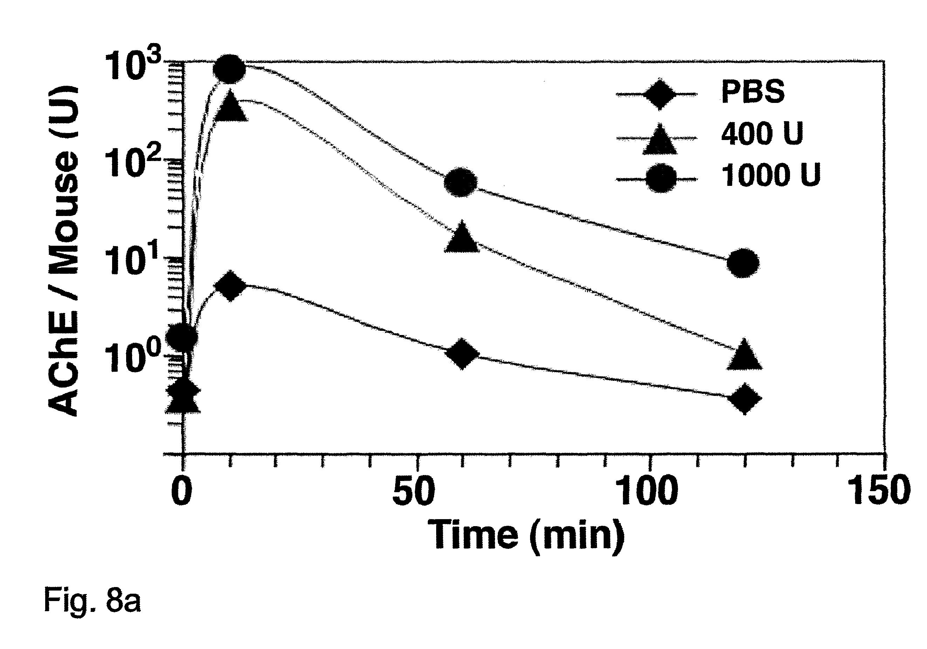

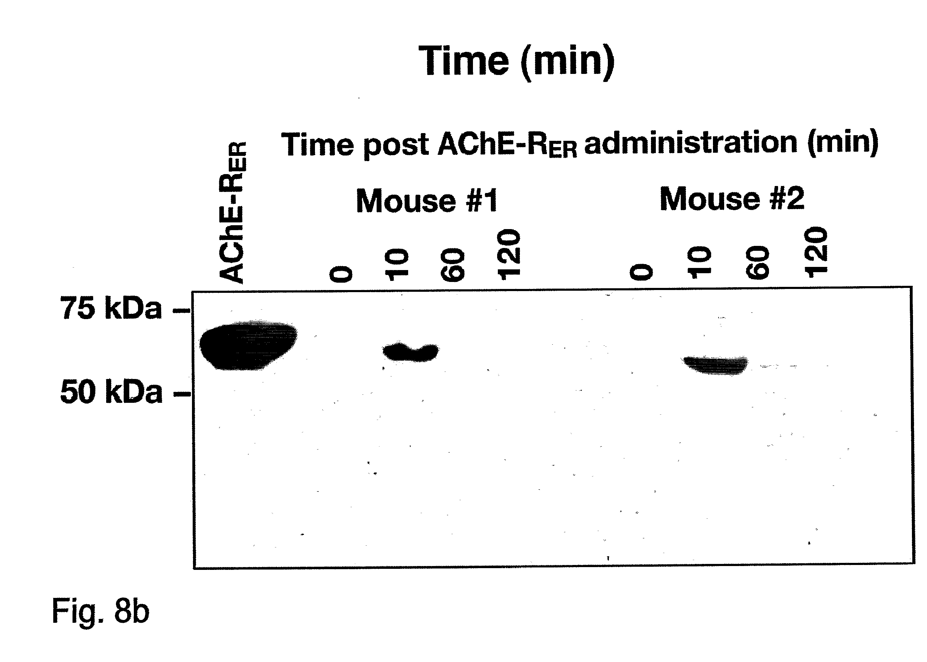

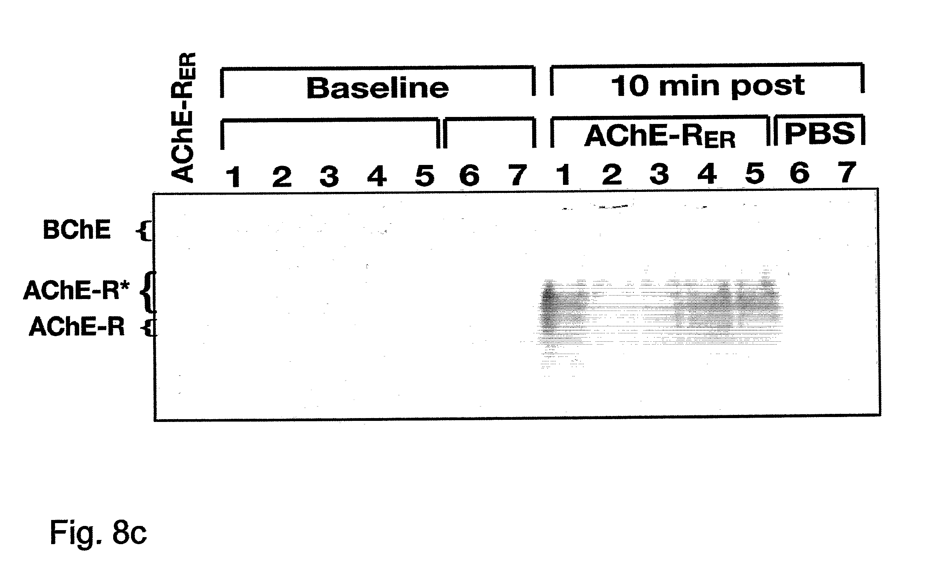

FIGs. 8A-C are graphs and photographs illustrating the parmacokinetics of plant-produced AChE-R_{ER}. Figure 8A is a clearance profile. Groups of 5 mice were injected with either 400U, 1000U of plant-produced AChE-R_{ER} or an equivalent volume of saline. Plasma samples were collected and assayed for AChE activity in the presence of Iso-OMPA, a selective BChE inhibitor. Figure 8B is an immunoblot detection of intact circulating AChE-R_{ER}. Plasma protein samples from 2 mice injected with 400U AChE-R_{ER} were resolved by SDS-PAGE and subjected to immunoblotting. Figure 8C: Circulating AChE-R_{ER} displays decreased migration in non-denaturing PAGE. Plasma samples from 5 mice injected with 400U AChE-R_{ER} and 2 mice injected with PBS were prepared and subjected to non-denaturing PAGE followed by staining of catalytically active AChE. Fast migrating AChE-R monomers appeared only following injection of the recombinant enzyme. In both Figures 8B and 8C, purified plant-produced AChE-R_{ER} was resolved alongside the serum samples for comparison.

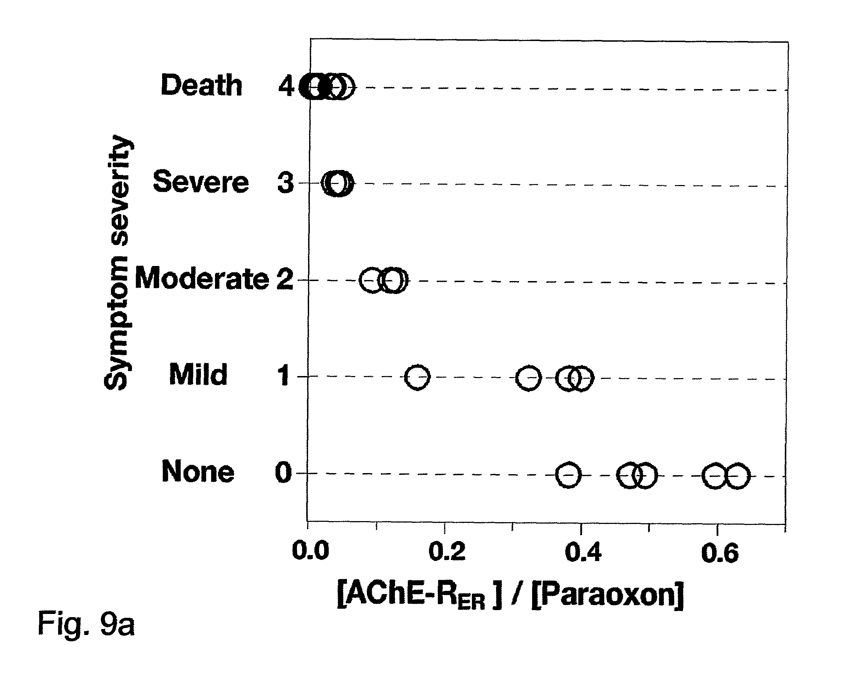

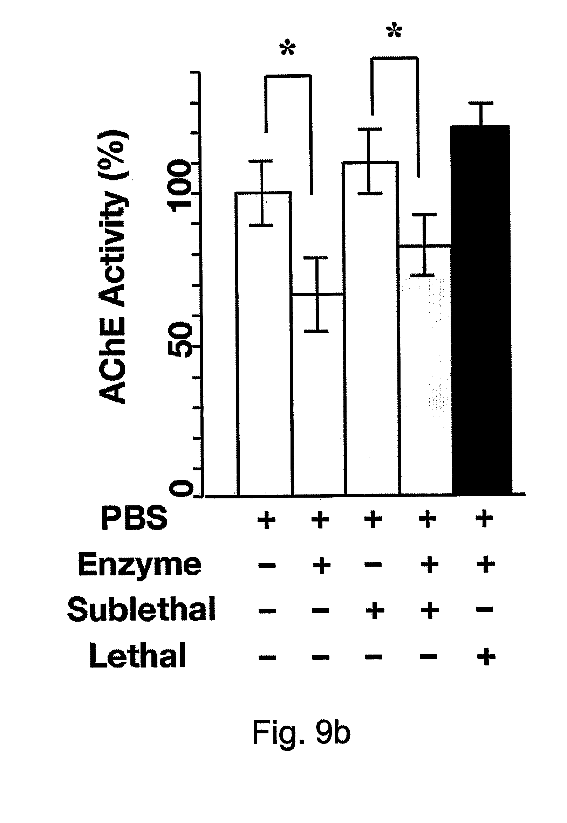

FIGs. 9A-B are graphs illustrating the activity of plant derived AChE-R_{ER} in vivo. Figure 9A illustrates that plant-derived AChE-R_{ER} can protect mice against lethal challenge of paraoxon. Mice were injected i.v. with plant-derived AChE-R_{ER} followed, after 10 min, by another i.v. injection of paraoxon (750 mg/kg) to yield the indicated enzyme/OP ratio. Each circle represents an individual mouse. Symptoms were scored as described in the text. Figure 9B illustrates that plant-derived AChE-R_{ER} and paraoxon can reciprocally modulate accumulation of murine AChE-R *in vivo.* Plasma samples were harvested 10 days post treatments and analyzed for their content of AChE activity. Asterisks indicate statistically significant changes (P<0.05, Student's t test).

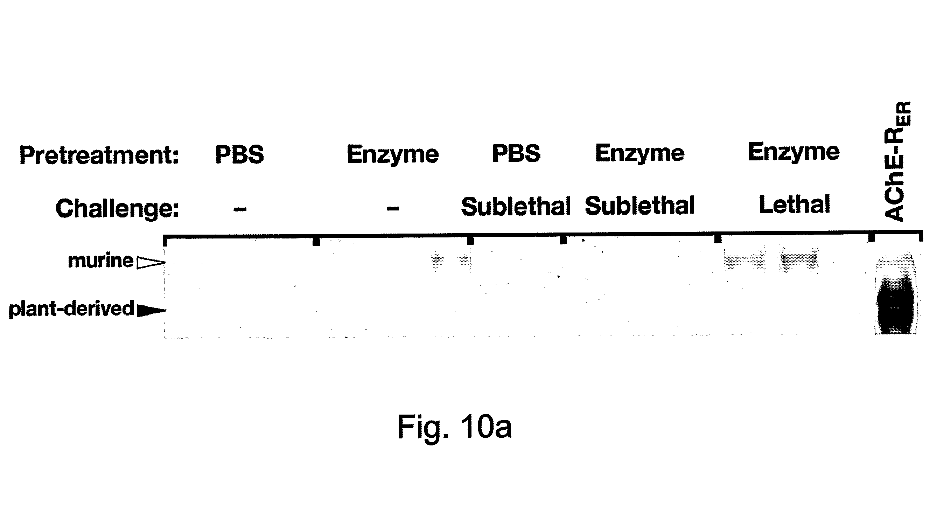

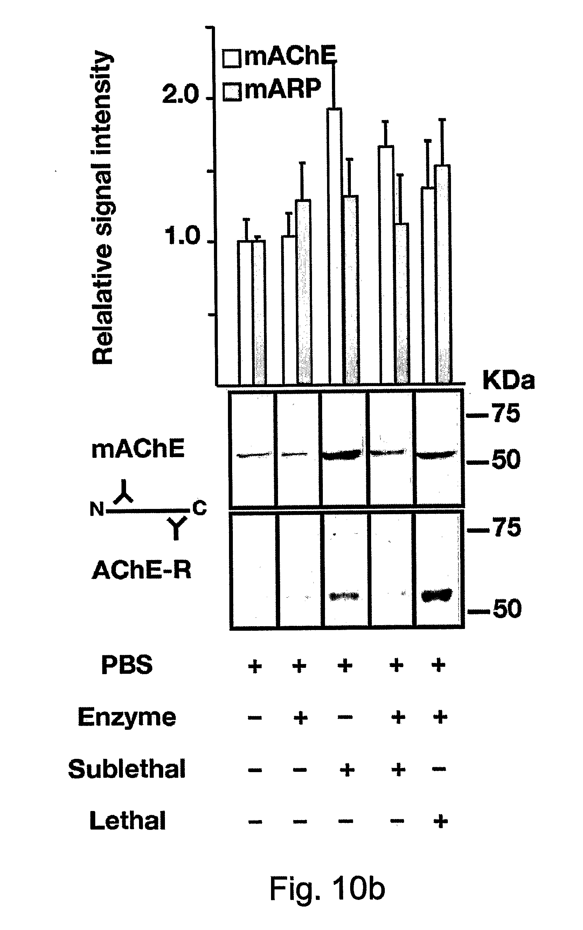

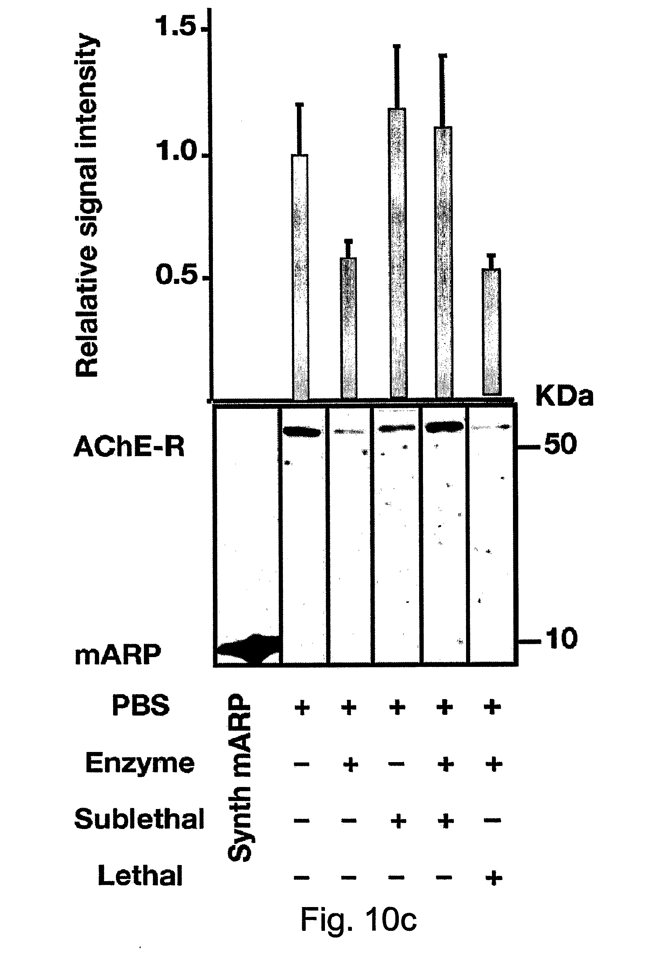

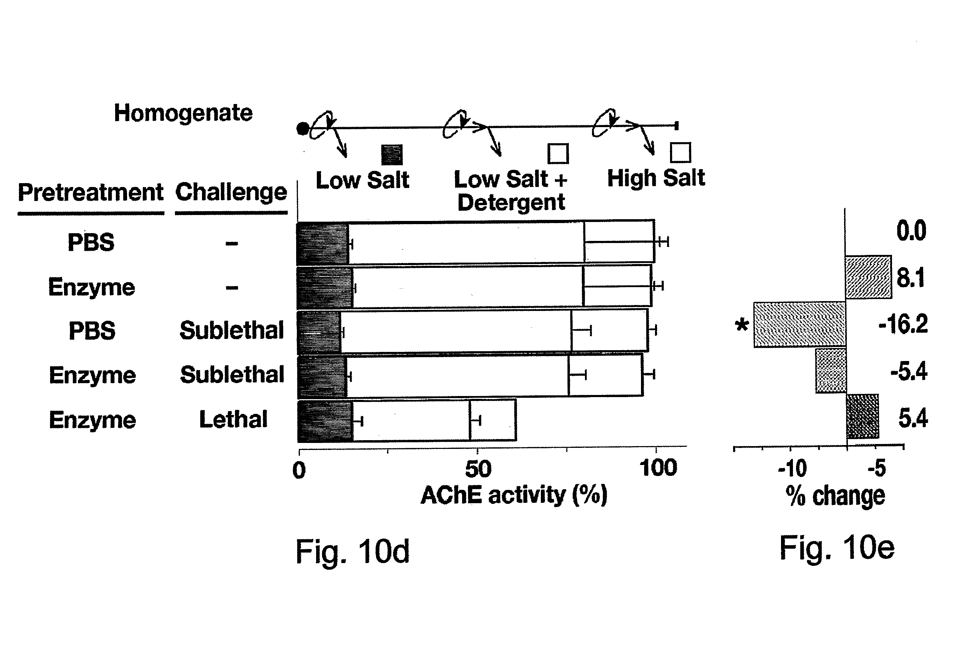

FIGs. 10A-E are graphs and photographs illustrating that plant-derived AChE-R_{ER} and paraoxon can reciprocally modulate accumulation of murine AChE-R *in vivo.* Samples of plasma (Figures 10A-B) and quadriceps muscles (Figures 10C-E) were harvested 10 days post treatments and were analyzed for their content of AChE protein and enzymatic activity. Figures 10A: Plasma proteins were resolved by non-denaturing PAGE followed by staining of active AChE. Plant-derived AChE-R_{ER} served for comparison. Figure 10B: Plasma proteins were resolved by SDS-PAGE followed by immunoblotting. Total AChE protein was detected with Abs directed to the common domain of all mouse AChE variants and murine AChE-R by Abs specific to its unique C-terminal domain (mARP). Representative blots are shown and above them densitormetric quantitation of band intensities (Analysis was conducted 5 times per sample, with 3-6 animals per group). Figure 10C: Muscle proteins were sequentially fractionated based on their solubility in Low Salt buffer (containing 144 mM NaCl. 50 mM MgCl₂ and 10 mM sodium phosphate, pH 7.4), Low Salt + Detergent (1 % Triton X-100), and finally High Salt (1 M NaCl and 10 mM sodium phosphate) as previously described [Lee et al., Jama 290, 659-662]. Proteins in the Low Salt + Detergent fraction were resolved by SDS-PAGE followed by immunoblotting and detection with the AChE-R specific Ab. Synthetic mARP peptide served as a positive control. Figure 10D: AChE activity was assayed in all three fractions (mean±SEM). Figure 10E: Percent change (relative to PBS control) of AChE activity in the Low Salt fraction. Asterisk denotes statistical significance (*P*<0.04, Student's t test).

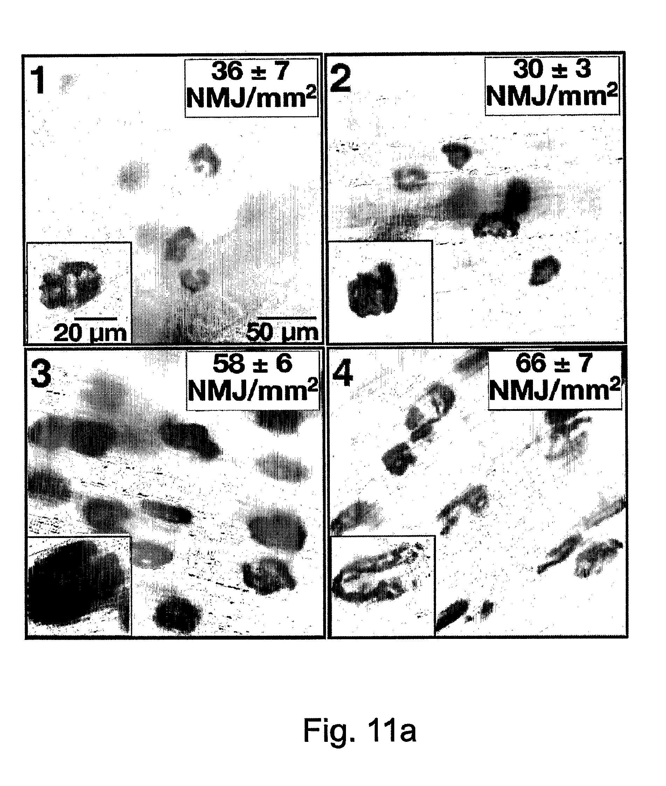

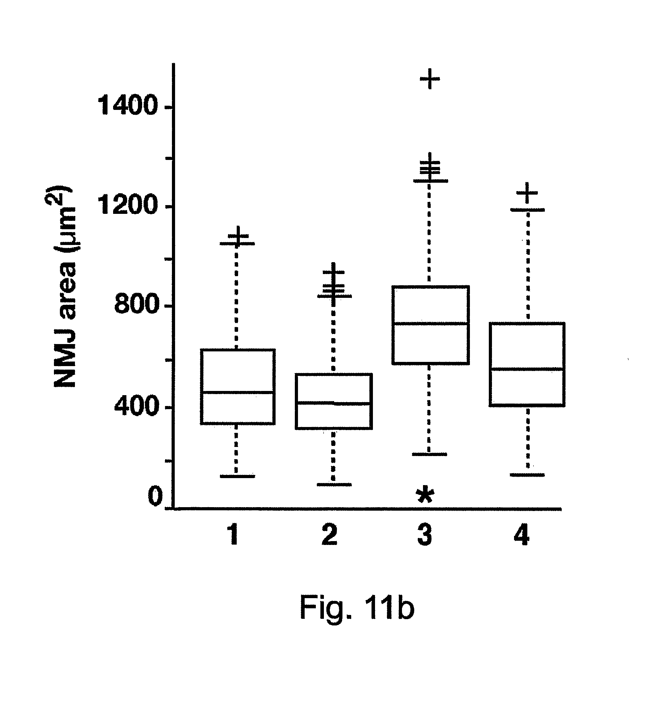

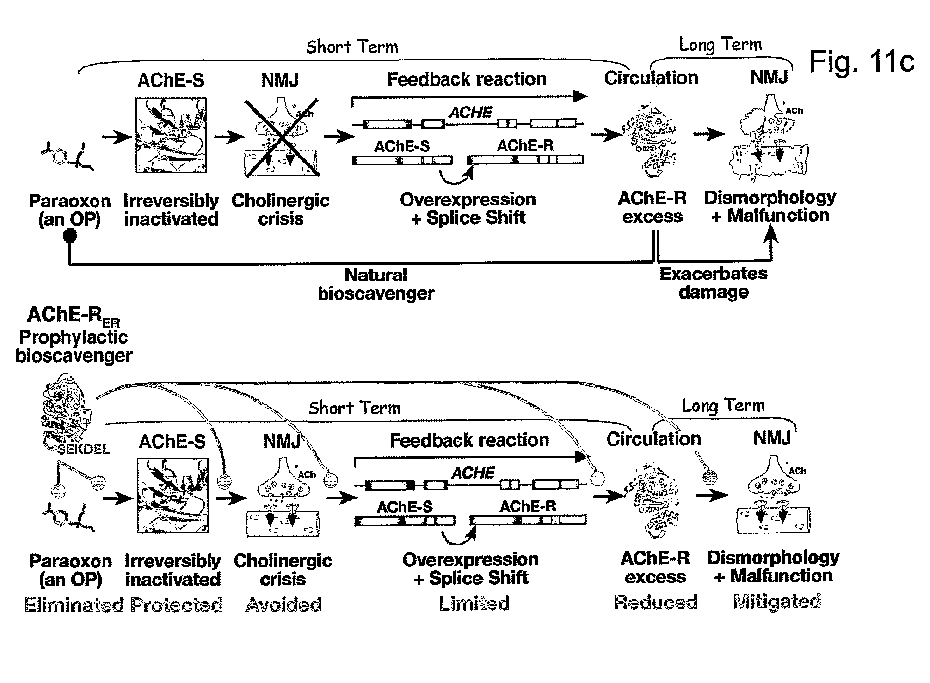

FIGs. 11A-C illustrate that AChE-R_{ER} pre-treatment ameliorates morphological NMJ damage induced by OP poisoning. Figure 11A: Mice were either treated with PBS (1), 400U AChE-R_{ER} (2), an 0.8 x LD₅₀ of paraoxon (3), or 400U AChE-R_{ER} and an 0.8 x LD₅₀ of paraoxon (4). 10 days post-treatment, diaphragm muscle was dissected and stained for catalytically active AChE to visualize the number and size of NMJ. NMJ density (NMJs / mm²) was measured and indicated by an average ± SEM within each section, al-a4. Figure 11B: Analysis of NMJ density and morphology. NMJ area (µm²) was box plotted using the MATLAB 7.0.1 software. The box is built from lines at the lower quartile, median and upper quartile values. The whiskers show the rest of the data and outliers indicated by +, present data beyond the whiskers. Asterisk indicates P<0.01 (median test). Both analyses suggest that paraoxon exposure increased both the density of NMJs and the fraction of large synapses (see also insets in Figure 11A(3) as compared to Figure 11A(1)). Pre-treatment with plant-derived AChE-R_{ER} prevented at least part of the effect on NMJ area, while plant-derived AChE-R_{ER} alone did not alter NMJ density or area within the non-exposed diaphragm. Figure 11C is a model for mechanism of protection from acute and chronic consequences of OP intoxication.

### DESCRIPTION OF THE PREFERRED EMBODIMENTS

The present invention is of materials and methods for treating or preventing organophosphate exposure associated damage, specifically, the present invention relates to the use of AChE-R for treating or preventing organophosphate exposure associated damage.

The principles and operation of the invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Exposure to organophosphates (OPs) in the form of nerve agents (e.g. sarin, soman and VX) and pesticides (e.g., paraoxon, parathion and malathion) may result in acute cholinergic effects by inhibition of acetylcholinesterase (AChE), permitting continuous firing of neurons and thereby producing toxicity, behavioral deficits and death. Recently, such agents pose an ever increasing military and civilian threat due to heightened terrorist activity.

While reducing the present invention to practice, the present inventors uncovered that AChE-R and its active portion ARP can be used for protecting against organophosphate exposure associated damage.

As is illustrated hereinbelow and in the Examples section which follows, exogenously applied AChE-R protected animals from an OP challenge. 6-8 week old male FVB/N mice were pretreated with increasing amounts of plant-produced human AChE-R (dose adjusted to body weight), followed by challenge with single-bolus 1.14 x LD₅₀ paraoxon (660 µg/kg) and symptoms were observed and recorded. This dose of paraoxon is fatal for otherwise untreated mice, with death preceding by severe cholinergic symptoms (convulsions and strained respiratory ability), occurring within 5-10 min. Pretreating 17 mice with plant-derived AChE-R reduced mortality even when the molar ratio of AChE-R/OP was as low as 0.04, far lower than the comparable stoichiometric protection by BChE [Raveh, L., Grunwald, J., Marcus, D., Papier, Y., Cohen, E., and Ashani, Y. (1993) Human butyrylcholinesterase as a general prophylactic antidote for nerve agent toxicity. In vitro and in vivo quantitative characterization. Biochem. Pharmacol. 45, 2465-2474]. At this low molar ratio, the surviving animals initially displayed severe symptoms followed by more moderate symptoms (less drastic convulsions and tremor). Symptoms gradually diminished and within 4 hours, mice exhibited only mild indications (decreased motor activity and general malaise). Importantly, all mice that survived the first 15 min following paraoxon injection showed apparently complete recovery within 24 hours.

At enzyme/agent molar ratios. >0.09, initial symptoms were moderate, and at >0.2 the initial symptoms were only mild. Mice treated with enzyme/agent ratio of >0.5 did not show any signs of post-exposure symptoms. Six-month monitoring of all surviving mice revealed no obvious neuropathy or other symptoms. Exceedingly low titers of IgGs specific to the administered human enzyme were observed; however neither AChE-specific IgEs nor auto-Abs directed against murine AChE² could be detected in serum samples from mice treated even with the largest dose of plant-derived AChE-R.

The present inventors have further shown that AChE-R protects from the delayed effects of OP poisoning. Mice were pre-treated by i.v. administration of PBS +/- AChE-R (400 U) followed by a sub-lethal (0.8 LD₅₀) or lethal (1.13 LD₅₀) paraoxon challenge. All exhibited severe cholinergic symptoms followed by recovery. (survival rates were 100% and 70% for sub-lethally- and lethally-challenged mice, respectively). Ten days post-treatment, mice were euthanized and plasma and skeletal muscle samples were harvested. At this time, all mice predictably experienced a small increase in their plasma AChE levels induced by the OP challenge. Plasma AChE activity at this delayed time point was substantially lower in enzyme-treated animals as compared to unprotected sub-lethally challenged mice, suggesting an effective yet incomplete offset of the long-lasting AChE-R over-production by administration of plant-derived AChE-R. Administration of plant-derived AChE-R alone significantly limited the levels of plasma AChE-R activity 10 days post injection, supporting the notion that this offset was a feedback response to the high levels of AChE molecules in the circulatory system. Furthermore, the enzyme-pretreatment modulation of the OP-induced increase in plasma AChE activity is mirrored by corresponding changes in AChE monomers, probably murine AChE-R, observed by non-denaturing gels of samples obtained 10 days post-challenge. The post-poisoning increase in the plasma levels of endogenous murine AChE, specifically murine AChE-R, can thus be at least partially mitigated by exogenously applied plant-derived enzyme.

Muscle AChE activities in AChE-R pre-treated mice were similar to those of unchallenged controls, demonstrating that the administered AChE-R served as a "decoy" protecting muscle AChE-R from inhibition while decreasing OP-induced AChE-R production in the mouse circulation.

Paraoxon exposure increased both the density and the fraction of large NMJs in intact diaphragm muscles dissected 10 days post-treatment, stained for catalytically active AChE as compared to the PBS control. Pre-treatment with 400U AChE-R prevented at least part of the exposure-induced enlargement of NMJ area, whereas treatment with 400U AChE-R alone did not alter NMJ density or area within the non-exposed diaphragm. These findings attribute the attenuated NMJ dismorphology effects to ameliorated feedback response in host muscle tissues.

In addition, the present inventors have also shown that administration of the natural cleavable C-terminal peptide of AChE-R, ARP to mice exposed to paraoxon, facilitated retrieval of motor activity and recovery of cognitive functions as determined by analyzing a number of parameters including AChE activity, corticosterone, body temperature and cognitive functions.

All these findings support the use of AChE-R for the treatment and prevention of organophosphate exposure associated damage.

Thus, according to an aspect of the invention, there is provided a method of treating or preventing organophosphate (OP) exposure associated damage in a subject in need thereof, the method comprising providing the subject with a therapeutically effective amount of AChE-R to thereby treat or prevent the organophosphate exposure associated damage in the subject.

As used herein the term "treating" refers to preventing, curing, reversing, attenuating, alleviating, minimizing, suppressing or halting the deleterious effects of the immediate life-threatening effects of organophosphate intoxication and its long-term debilitating consequences.

As used herein the phrase "organophosphate exposure associated damage" refers to short term (e.g., minutes to several hours post- exposure) and long term damage (e.g., one week up to several years post- exposure) to physiological function (e.g., motor and cognitive functions). Organophosphate exposure associated damage may be manifested by the following clinical symptoms including, but not limited to, headache, diffuse muscle cramping, weakness, excessive secretions, nausea, vomiting and diarrhea. The condition may progress to seizure, coma, paralysis, respiratory failure, delayed neuropathy, muscle weakness, tremor, convulsions, permanent brain dismorphology, social/behavioral deficits and general cholinergic crisis (which may be manifested for instance by exacerbated inflammation and low blood count. Extreme cases may lead to death of the poisoned subjects.

As used herein the term "organophosphate compound" refers to a compound comprising a phosphoryl center, and further comprises two or three ester linkages. In some aspects, the type of phosphoester bond and/or additional covalent bond at the phosphoryl center classifies an organophosphorus compound. In embodiments wherein the phosphorus is linked to an oxygen by a double bond (PdbdO), the OP compound is known as an "oxon OP compound" or "oxon organophosphorus compound." In embodiments wherein the phosphorus is linked to a sulfur by a double bond (PdbdS), the OP compound is known as a "thion OP compound" or "thion organophosphorus compound."

Additional examples of bond-type classified OP compounds include a phosphonocyanate, which comprises a P--CN bond; a phosphoroamidate, which comprises a P--N bond; a phosphotriester, which comprises a P--O bond; a phosphodiester, which comprises a P--O bond; a phosphonofluoridate, which comprises a P--F bond; and a phosphonothiolate, which comprises a P--S bond. A "dimethyl OP compound" comprises two methyl moieties covalently bonded to the phosphorus atom, such as, for example, malathion. A "diethyl OP compound" comprises two ethoxy moieties covalently bonded to the phosphorus atom, such as, for example, diazinon.

In general embodiments, an OP compound comprises an organophosphorus nerve agent or an organophosphorus pesticide.

As used herein, a "nerve agent" is an inhibitor of a cholinesterase. The toxicity of an OP compound depends on the rate of release of its phosphoryl center (e.g., P--C, P--O, P--F, P--S, P--CN) from the target enzyme. Preferred nerve agents are inhibitors of a cholinesterase (e.g., acetyl cholinesterase) whose catalytic activity is often critical for health and survival in animals, including humans.

Certain OP compounds are so toxic to humans that they have been adapted for use as chemical warfare agents (CWAs), such as tabun, soman, sarin, cyclosarin, VX, and R-VX. A CWA may be in airborne form and such a formulation is known herein as an "OP-nerve gas." Examples of airborne forms include a gas, a vapor, an aerosol, a dust, or a combination thereof. Examples of an OP compounds that may be formulated as an OP nerve gas include tabun, sarin, soman, VX, GX, or a combination thereof. An example of an organophosphate which is close to, albeit not similar in its properties to those of the nerve gases is that of DFP, diisopropylfluorophosphonate, which is considerably less volatile than the other members of this group.

In addition to the initial inhalation route of exposure common to such agents, CWAs, especially persistent agents such as VX and thickened soman, pose threats through dermal absorption [In "Chemical Warfare Agents: Toxicity at Low Levels," (Satu M. Somani and James A. Romano, Jr., Eds.) p. 414, 2001]. Such persistent CWA agents remain as a solid or liquid while exposed to the open air for more than three hours. Often after release, a persistent agent may convert from an airborne dispersal form to a solid or liquid residue on a surface, thus providing the opportunity to contact the skin of a human.

Examples of an OP pesticide include bromophos-ethyl, chlorpyrifos, chlorfenvinphos, chlorothiophos, chlorpyrifos-methyl, coumaphos, crotoxyphos, crufomate, cyanophos, diazinon, dichlofenthion, dichlorvos, dursban, EPN, ethoprop, ethyl-parathion, etrimifos, famphur, fensulfothion, fenthion, fenthrothion, isofenphos, jodfenphos, leptophos-oxon, malathion, methyl-parathion, mevinphos, paraoxon, parathion, parathion-methyl, pirimiphos-ethyl, pirimiphos-methyl, pyrazophos. quinalphos, ronnel, sulfopros, sulfotepp, trichloronate, or a combination thereof.

As used herein the phrase "a subject in need thereof' refers to a human or animal subject who is sensitive to OP toxic effects. Thus, the subject may be exposed or at a risk of exposure- to OP. Examples include civilians contaminated by a terrorist attack at a public event, field workers subjected to pesticide/insecticide OP poisoning, truckers who transport pesticides, pesticide manufacturers, dog groomers who are overexposed to flea dip, pest control workers and various domestic and custodial workers who use these compounds, military personnel exposed to nerve gases.

As mentioned, in some embodiments of the invention the method is effected by providing the subject with a therapeutically effective amount of AChE-R.

As used herein the term "AChE-R" refers to the acetylcholinesterase readthrough variant such as set forth in GenBank Accession Number DQ 140347 (e.g., SEQ ID NOs. 2) or an active portion thereof e.g., ARP set forth in SEQ ID NOs. 6 and 8.

The AChE-R of the present invention may be naturally expressed (i.e., purified), synthetic or recombinantly produced such as in bacteria, yeast, cell-lines, transgenic animal (e.g., see U.S. Pat. No. 5,932,780, herein incorporated by reference in its entirety) or plants (moss, algae, monocot or dicot, as well as other plants. Examples include, but are not limited to, leafy crops, oil crops, alfalfa, tobacco, tomatoes, bananas, carrots, lettuce, maize, cucumber, melon, potatoes grapes and white clover.

The plant cell may optionally be any type of plant cell such as a plant root cell (i.e. a cell derived from, obtained from, or originally based upon, a plant root), more preferably a plant root cell selected from the group consisting of, a celery cell, a ginger cell, a horseradish cell and a carrot cell. see e.g., Examples section, expression in tobacco).

Methods of producing recombinant proteins are well known in the art. Specific examples for recombinant protein production in plant cells are provided in PCT WO2005/080544, herein incorporated by reference in its entirety.

AChE-R of some embodiments of the invention may be chemically modified for increasing bioavailability. Methods of protein PEGylation are well known in the art.

As OP can be rapidly absorbed from lungs, skin, gastro-intestinal (GI) tract and mucous membranes, AChE-R may be provided by various administration routes or direct application on the skin.

For example, AChE-R may be immobilized on a solid support e.g., a porous support which may be a flexible sponge-like substance or like material, wherein the AChE-R is secured by immobilization. The support may be formed into various shapes, sizes and densities, depending on need and the shape of the mold. For example, the porous support may be formed into a typical household spong, wipe or a towelette.

For example, such articles may be used to clean and decontaminate wounds, while the immobilized AChE-R will not leach into a wound. Therefore, the sponges can be used to decontaminate civilians contaminated by a terrorist attack at a public event.

Alternatively or additionally, AChE-R may be administered to the subject per *se* or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the AChE-R accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition, which is incorporated herein by reference.

Suitable routes of administration may, for example, include oral, rectal, transmucosal, especially transnasal, intestinal or parenteral delivery, including intramuscular, subcutaneous and intramedullary injections as well as intrathecal, direct intraventricular, intravenous, inrtaperitoneal, intranasal, or intraocular injections.

Alternately, one may administer the pharmaceutical composition in a local rather than systemic manner, for example, via injection of the pharmaceutical composition directly into a tissue region (e.g., skin) of a patient.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically. acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution. Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuos infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of the present invention may also be formulated in rectal compositions such as suppositories or retention enemas, using, e.g., conventional suppository bases such as cocoa butter or other glycerides.

Pharmaceutical compositions suitable for use in context of the present invention include compositions wherein the active ingredients are contained in an amount effective to achieve the intended purpose. More specifically, a therapeutically effective amount means an amount of active ingredients (nucleic acid construct) effective to prevent, alleviate or ameliorate symptoms of a disorder (e.g., ischemia) or prolong the survival of the subject being treated.

Determination of a therapeutically effective amount is well within the capability of those skilled in the art, especially in light of the detailed disclosure provided herein.

For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide plasma or brain levels of the active ingredient are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Since AChE-R sequesters OP molecules, stoichiometric quantities may be administered, essentially, one enzyme monomer for each poison molecule.

AChE-R may be administered prior to the OP exposure (prophylactically, e.g., 10 or 8 hours before exposure), and alternatively or additionally administered post exposure, even days after (e.g., 7 days) in a single or multiple-doses.

Without being bound to theory, it is suggested that the provision of AChE-R immediately after exposure (which is recommended to occur within two hours from the event, to avoid irreversible phenomena), will arrest or minimize over- production of endogenous AChE-R in the delayed phase, protecting the neuromuscular junctions.

Embodiments of the invention also contemplate the use of other agents in combination with AChE-R for the treatment or prevention of OP damage.

Thus, according to an exemplary embodiment, AchE-R may be administered by inhalation to protect the lungs and injection (i.v.) to protect the circulation up to 2 hours post exposure. Atropine may be added 2-4 hours post exposure. Daily injections of AChE-R may be administered up to 7 days post poisoning. Oximes like H1-6 and mono-bisquaternary oximes such as pralidoxime chloride (2-PAM) may be added to improve treatment efficacy.

Compositions of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as if further detailed above.

The ability of AChE-R to sequester OP molecules, suggests use of same in the decontamination of OP contaminated surfaces and detoxification of airborne OP.

Thus, an aspect of the invention further provides for a method of detoxifying a surface contaminated with an OP molecule; or preventing contamination of the surface with OP. The method is effected by contacting the surface with AChE-R.

Thus, synthetic and biological surfaces contemplated according to embodiments of the invention include, but are not limited to, equipment, laboratory hardware, devices, fabrics (clothes), skin (as described above) and delicate membranes (e.g., biological). The mode of application will very much depend on the target surface. Thus, for example, the surface may be coated with foam especially when the surface comprises cracks, crevices, porous or uneven surfaces. Application of small quantities may be done with a spray-bottle equipped with an appropriate nozzle. If a large area is contaminated, an apparatus that dispenses a large quantity of foam may be utilized.

Coatings, linings, paints, adhesives sealants, waxes which may comprise the AChE-R may be applied to the surface. Exemplary embodiments for such are provided in U.S. Pat. Application No. 20040109853.

Surface decontamination may be further assisted by contacting the surface with a caustic agent; a decontaminating foam, a combination of baking condition heat and carbon dioxide, or a combination thereof.

In addition to the above described coating compositions, OP contamination may be prevented or detoxified using an article of manufacture which comprise the AChE-R immobilized to a solid support in the form of a sponge (as described above), a wipe, a fabric and a filter (for the decontamination of airborne particles). Chemistries for immobilization are provided in U.S. Pat. Application 20040005681.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion. Reference is now made to the following examples, which together with the above descriptions, illustrate the invention in a non limiting fashion.

Generally, the nomenclature used herein and the laboratory procedures utilized in the present invention include molecular, biochemical, cellular and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); all of which are incorporated by reference as if fully set forth herein. Other general references are provided throughout this document. The procedures therein are believed to be well known in the art and are provided for the convenience of the reader. All the information contained therein is incorporated herein by reference.

### GENERAL MATERIAL AND METHODS FOR EXAMPLES 1-4

***Establishment of stable cell lines over-expressing specific AChE variants:*** CHO cells were grown as described (Perry et al. 2004, Neoplasia 6, 279-86). Transfection involved the AChE-R SEQ ID NO: 1) and AChE-S (SEQ ID NO: 15) encoding cDNAs (Soreq and Seidman 2001, Nat Rev Neurosci 2, 294-302) as well as cDNA encoding green fluorescent protein (GFP; Perry et al. 2002, Oncogene, in press) and followed standard selection procedures.

***In vivo* experiments:** 7-8 weeks CD-1 male mice were housed in a specific pathogen free (SPF) environment (maximum 6 animals per cage). Animals were provided ad libitum, a commercial rodent diet (Harlan Teklad TRM Rat/Mouse Diet, Rehovot, Israel) and free access to drinking water. Automatically controlled environmental conditions were set to maintain temperature at 20-24 °C with a relative humidity of 30-70 %, a 12-hr light/ 12-hr dark cycle and 10-30 air changes/hr in the study room. Two weeks prior to the test and following overnight food deprivation, biotelemetric transmitters were implanted in the peritoneal cavity of the tested mice under general anesthesia injected intraperitoneally (i.p.) (Cohen et al. 2002, Mol Psychiatry 7, 874-85). Following implantation, mice were housed in separate cages with free access to food and water. 2-weeks following recovery from the surgical procedure, body temperature (BT) and motor activity (MA), were recorded pre- and post-treatments (Cohen et al. 2002, Mol Psychiatry 7, 874-85). Two days following pre-treatment and the establishment of baseline values, mice were treated under continuous recording. Treatments included a first single i.p. injection of saline, 0.4 mg/kg paraoxon (Sigma-Aldrich, Rehovot, Israel), or 2.5 mg/kg *E. coli* LPS (Sigma-Aldrich, Rehovot, Israel). One hour following first injection and 1x daily throughout the following 5 days of the experiment, mice were injected intravenously (second injection, i.v.) with 0.25 mg/kg of mARP (Nijholt *et al.* 2004) or saline as control. Animals were periodically observed for clinical signs and changes in body weight during the study. At the end of the study, animals were euthanized by CO₂ asphyxiation and plasma and tissue samples were collected. Plasma and tissues from 3 non-implanted untreated naïve mice served for controls. All tested euthanized animals were subjected to full necroscopy. The Hebrew University's committee for animal experiments approved this study.

***AChE activity and corticosterone assays:*** AChE activities were measured in animal plasma using multiwell plate-adapted Ellman's assay (Seidman et al. 1995, Mol Cell Biol 15, 2993-300) in the presence of 50 µM of the BChE specific inhibitor tetraisopropylpyrophosphoramide (Iso-OMPA). Corticosterone levels in the plasma were measured using the OCTEIA Corticosterone kit (IDS Ltd, Boldon, UK) according to the manufacture's instructions.

***Real-time RT-PCR:*** To test for the consequences of tested treatments on AChE gene expression in skeletal muscle, total RNA was purified from frozen skeletal muscle removed from euthanized treated and control mice using the RNeasy mini kit (Qiagen, Valencia, CA) according to the instructions for fibrous tissues. RNA was subjected to real-time RT-PCR quantification using the Light Cycler system (Roche Diagnostics, Mannheim, Germany). Two sets of primers were used to amplify both the common domain of AChE mRNA (273(+) GGTAGACGGGGACTTC (SEQ ID NO: 9) / 637(-) ATGTAGGCATAGACCCG (SEQ ID NO: 10) and the 3' region that is unique for AChE-S mRNA (637(+) CGGGTCTATGCCTACATC (SEQ ID NO: 11) / 1250(-) GCTCGGTCGTATTATATCCCA (SEQ ID NO: 12). Values were calibrated to those of the standard transcript, β-actin mRNA (Meshorer et al. 2005, Faseb J 19, 910-22).

***Immunoblot:*** Immunoblots were performed as described (Birikh et al. 2003, Proc Nat1 Acad Sci U S A 100, 283-8) using an antibody targeted to the common region of the AChE protein (N-19, Santa Cruz Biotechnology, Santa Cruz, CA).

### EXAMPLE 1

### Establishment of stable cell lines over-expressing specific AChE variants

### RESULTS

CHO cell lines stably co-expressing GFP with either human AChE-S or AChE-R, all under control of the minimal CMV promoter-enhancer were established and tested for the corresponding hydrolytic activities of the expressed enzyme. In both cases, viable cell lines were obtained with appreciable activity levels. CHO cells expressing only GFP served as control. Several cell lines were established for each of these vectors, Figure 1B presents representative examples of the expression and secretion levels of AChE variants in such cell lines.

Both AChE-R and AChE-S activities appeared in the intracellular and the secreted fractions of the established cell lines. Acetylthiocholine hydrolysis levels increased from negligible levels in GFP-expressing cells up to the levels of micromole substrate hydrolyzed per mg cellular protein or ml medium (Figure 1C), higher than transiently transfected cells (Velan et al. 1991, Cell Mol Neurobiol 11, 143-56) but lower than plant expression (Geyer et al. 2005, Chem Biol Interact.). Predictably, AChE-R tended to be more efficiently secreted than AChE-S, and showed two discernable bands in Western blots, suggesting cleavage to yield free hARP (Cohen et al. 2003, J Mol Neurosci 21, 199-212). AChE-S as well was found to be subject to such cleavage (Figure1B and (Sternfeld et al. 1998, J Neurosci 18, 1240-9)). Stably transfected cell lines showed unmodified doubling time as compared to the parent cell line, and MTT viability tests demonstrated the lack of apoptosis (data not shown).

### EXAMPLE 2

### Systemic effects on plasma AChE activities and corticosterone levels

Exposure to OP agents results in a reduced body temperature and motor activity, similar to the consequences of acute mammalian stress responses (Karczmar 2000, E. Giacobini, ed., pp. 157-180. Martin Dunitz, London; Marrs 1993, Pharmacol Ther 58, 51-66). At least some of those effects are caused by the blockade of AChE catalytic activity, leading to increase in ACh and cortisol levels (Kaufer et al. 1998, Nature 393, 373-7; Kaufer et al 1999, Chem Biol Interact 119-120, 349-60) and to inherent cleavage of mouse (m)ARP (Dori et al. 2005, Cereb Cortex 15, 419-30; Grisaru et al. 2001, Mol Med 7, 93-105). To search for a potential capacity of mARP to interfere with AChE overproduction following organophosphate exposure, CD-1 mice were injected with saline, paraoxon, or *E. coli* lipopolysaccharide (LPS). Daily throughout the 5 days of the experiment, the mice were injected intravenously (i.v.) with 0.25 mg/kg of mARP or saline as control.

### RESULTS

AChE specific activities were measured in plasma of all animals, at the end of the 1 week experiment, in the presence of 50 µM ISO-OMPA (an inhibitor of butyrylcholinesterase (BChE), the main circulation cholinesterase). Paraoxon treatment caused an increase of plasma AChE activities which was sustained throughout the post-exposure week and was predictably higher in animals treated with mARP, compatible with previous findings for mARP (Grisaru et al. 2001. Mol Med 7, 93-105) and hARP (Grisaru et al. 2006, J Immunol 176, 27-35). Figure 2A presents this outcome of the mARP treatment against paraoxon poisoning. The changes in plasma AChE activities were accompanied by 3-fold increases in plasma corticosterone levels, under double saline treatment as compared to naïve and 5-fold increases under paraoxon followed by saline as compared to saline + saline. mARP ameliorated these effects and completely abolished the increased corticosterone levels (Figure 2B). Thus, administered mARP enhanced AChE gene expression yet prevented excessive corticosterone production post-OP exposure.

### EXAMPLE 3

### AChE-R manipulations following exposure affect host AChE gene expression

### RESULTS

Following a one week treatment, mRNA levels of the different AChE transcripts were measured in skeletal muscles of three of the treated as compared to three naive animals using the Light Cycler system for real-time RT-PCR quantification. Two sets of primers were employed to detect both the common domain of AChE mRNA (Figure 3B) and the 3' region that is unique for AChE-S mRNA (Figure 3C). Values were calibrated to those of the standard transcript, β-actin mRNA. Paraoxon caused a 2-fold decrease in AChE mRNA, accompanied by an almost 8-fold decrease in AChE-S mRNA levels as compared to non-treated naive mice. This is compatible with the notion of a combined muscle reaction involving facilitated destruction of pre-existing AChE-S mRNA, transcriptional activation and a shift in alternative splicing yielding nascent AChE-R mRNA. mARP obliterated the paraoxon-induced decrease in both AChE mRNA regions, suggesting that it interfered with both the transcriptional activation and the alternative splicing shift of AChE pre-mRNA. Differences between saline and mARP treated paraoxon-exposed animals was observed both in total and in AChE-S mRNA, indicating that it reflects a real change. Thus, by 7 days post-exposure, mARP increased post-exposure AChE production in blood and prevented the post-exposure decrease in muscle AChE production.

### EXAMPLE 4

### mARP administration alters the physiological consequences of exposure to paraoxon or bacterial LPS.

The potential post-challenge activities of mARP were further explored using the above treated animals by recording body temperature (BT) and motor activity (MA), pre- and post-treatments using transmitter implanted-mice (Cohen et al. 2002, Mol Psychiatry 7, 874-85).

### RESULTS

Recording began 2-weeks following recovery from the surgical procedure. Two days after establishing baseline values, mice were treated under continous recording. Saline administration alone did not significantly change BT or MA values in the transmitter implanted mice as compared to naive (data not shown).

The inventors working hypothesis predicted that AChE-R accumulation following stress, LPS or anticholinesterase exposure should modulate the response to such insults (Romanovsky et al. 2005, Front Biosci 10, 2193-216). Administration of paraoxon, an anticholineaterase organophosphate, predictably caused a reduction in light and dark-phase BT and MA in the first day post-treatment. Figures 4A-F and 5A-F, respectively, demonstrate single animal examples for this reaction, followed by a partial recovery in BT following 6 hours. Figures 4A-F illustrate that MA remained low throughout the 6 days post-exposure; elevation of AChE-R levels by synthetic mARP injections somewhat limited the decrease in BT during the first day post-treatment and elevated BT during the following dark phase. As soon as day 1 post-saline exposure, mARP increased locomotor activity during both light and dark phases of the MA. By days 4,5 post- OP exposure, mARP injection also induced a dramatic increase in MA (Figures 5D-F). In the long-term, therefore, the employed regimen of mARP treatment increased light and dark-phase MA above normal values. The inflammatory stressor, LPS, reduced both BT and MA, albeit less than paraoxon (Figures 4A-F and 5A-F, respectively). On the first day post-LPS exposure, mARP did not modify BT and / or MA values, while in the long term, mARP increased light and dark-phase MA above nomal values. This is compatible with the symptoms recorded for AChE-R overexpressing transgenic mice following a cyrcadian shift (Cohen et al. 2002, Mol Psychiatry 7, 874-85) and consistent with the AChE mRNA and protein production in muscle and plasma, respectively.

### CONCLUSION FOR EXAMPLES 1-4

Examples 1-4 aimed at clarifying three key issues related to the intricate AChE-R response to chemical and biological insults: firstly, to find out if the post-insult accumulation of AChE does not induce cell death. That CHO cells stably expressing either AChE-S or AChE-R could be established supports the notion that neither of these variants, at the measured expression levels, causes cell death.

Secondly to find out if overexpressed AChE exacerbates the damages caused by the tested insults or, inversely, acts to protect the affected organism from post-exposure consequences. The present findings argue in favor of the latter option, although the possibility of AChE-R-associated damages cannot be excluded. Thirdly, to identify AChE-R domain(s) responsible for these effects and to test a synthetic version of that domain for its capacity to mimic the ACHE-R protective effects. Yet more specifically, the present inventors examined a putative function for murine AChE-R overproduction and mARP in augmenting the body's defense following exposure to anti-cholinesterases or bacterial infection. The effects of injected mARP on plasma AChE activity and corticosterone levels, on body temperature and motor activity and on muscle AChE gene expression following paraoxon and LPS exposures all support the hypothesis that these changes act as mediators of the post-exposure events.

That intraperitoneally injected mARP affected BT, MA, blood AChE activity and muscle AChE mRNA levels strongly supports the notion that this naturally cleavable peptide is responsible for much of the previously observed effects attributed to AChE-R; furthermore, these findings raise the possibility of using synthetic ARP to treat exposed subjects for facilitating recovery and minimizing exposure-induced damages.

Organophosphate AChE inhibitors induce over-expression of AChE-R in brain, muscle and intestine. In the short-term, the shift in alternative splicing of AChE from AChE-S to the AChE-R variant clears the excess of ACh which accumulates under anti-AChE exposure. In the long-term, excess of soluble monomeric AChE-R can further induce hematopoietic cellular processes through its non-catalytic properties (e.g. granulocytosis and thrombopoiesis). In farmers, exposure to organophosphate insecticides was correlated to a higher risk for leukemia as well as with electroencephalographic and memory impairments. AChE is expressed in hematopoietic cell lineages and regulates the peripheral cholinergic status. The detailed mechanism(s) through which AChE controls such processes likely involve the recruitment of the scaffold protein RACK 1 and protein kinase (PK) C.

AChE-R is induced in CD34⁺ hematopoietic progenitors in culture following cortisol treatment. This elevation was associated with a cleavage of the AChE-R C-terminal peptide, human (h) ARP. The peptide cleavage may expose the truncated AChE to protease activity in the blood and occurs in human serum following exposure to LPS. Thus, following LPS administration and up-regulation of AChE-R, ACh levels in the blood decrease, but only transiently due to subsequent AChE degradation. Modulatory ACh levels further control pro-inflammatory cytokine production, a major response to acute stress. Ex-vivo administration of a synthetic peptide consisting of the 26 amino acid of hARP promotes expansion and differentiation of hematopietic stem cells, a pivotal element of organismal reactions to poisonous substances. Antisense suppression of AChE-R expression prevented this proliferative activity of ARP, suggesting that ARP administration promotes AChE gene expression, yielding auto-regulatory overproduction of its pre-protein, AChE-R. Consistent with the proliferative properties of hARP in hematopietic cells, the mouse C-terminal sequence of AChE-R, mARP, was shown to control migration and proliferation vs. differentiation processes in the developing cortex. Moreover, when injected to the lateral ventricle of the brain, mARP penetrated both to adjacent neuronal cells and to areas distant from the injection site, proving its capacity to undergo endocytosis and retrograde transport. Administration of mARP further enhanced fear memory and LTP in the treated mice, which could be suppressed by antisense treatment and was associated with hippocampal increase in PKCβII under over-expression of AChE-. Thus the use of this peptide should be experimented with caution due to its bimodal effects.

In both brain and blood, AChE-R interacts with the scaffold protein RACK1 and its partner PKCβII (Birikh *et al.* 2003; Sklan *et al.* 2006), which participate in a wide range of signal transduction pathways. The mARP peptide is capable of penetrating cells in live tissue. Within cells of both brain and blood origin, mARP interacts with the scaffold RACK1 protein, an intracellular carrier of protein kinase C βII. Formation of AChE-R/ RACKl/ PKCβII complexes reduces the cytoplasmic levels of free RACK1, interfering with its capacity to inhibit translation by interacting with ribosomal complexes. This should exert significant effects on cellular metabolism. AChE-R also accumulates after exposure to the immune stressor LPS, (Cohen *et al.* 2003; Pick *et al.* 2006) or in the serum of patients with the autoimmune disease myasthenia gravis (MG) and in serum and muscle of experimental autoimmune MG rats (EAMG) (Brenner *et al.* 2003). Antisense suppression of AChE-R prevented this accumulation and improved muscle function and clinical status of EAMG rats. In lumbar spinal cord of primates, antisense suppression of the stress-associated AChE-R diminished its accumulation in interneurons that synapse on motoneurons and reduced the cholinergic load on motoneuron. The primate and rodent studies performed are likely to be relevant for humans as well. However, tetracycline-controlled suppression of post-stress AChE-R production by a transgenic antisense sequence increased the risk of stress-associated catalepsy in the conditional transgenic mice. Together, this suggests a "safety window" of AChE-R and ARP levels which is compatible with appropriate motor and cognitive functions.

### GENERAL MATERIAL AND METHODS FOR EXAMPLES 5-11

***Engineering of N. benthamiana plants expressing human AChE-R:*** A plant-expression optimized DNA construct encoding human AChE-R (Genbank # DQ 140347) fused to the C-terminal ER-retention signal SEKDEL was synthesized de-novo, cloned into a plant expression vector and used for Agrobacterium tumefaciens mediated transformation of N. benthamiana (Mor, T. S., et al., Biotechnol. Bioeng. 75, 259-266). The plant line with the highest AChE activity was selected for propagation, seed production and further analysis (Geyer et al., 2005, Chem Biol Interact 157-158, 331-334).

***In vivo experiments:*** Circulatory t_{½} of plant-derived AChE-R_{ER} was determined in male 6-8 week-old FVB/N mice that were lightly anesthetized with Isoflurane (Rhodia, Bristol, UK), and placed in a restrainer prior to the treatment. Mice were i.v. injected (tail vein) with 80 µl saline containing 400 or 1000 units (U) of purified AChE-R_{ER}. At the indicated time points, blood samples (10-30 µl) from the tail vein were collected into 4 µl of 0.25 M EDTA in PBS and were kept on ice until separation of plasma by centrifugation (500 rpm for 30 min, 4 °C). Paraoxon LD₅₀ values for FVB/N mice were determined by bolus i.v. (tail vein) injections of aqueous solutions of paraoxon (Sigma) at different concentrations.

***Protection assays:*** Mice were injected with 0-10,000 U of AChE-R_{ER} in 100 µl of 0.9 % saline. ChE levels were determined from blood samples drawn 10 min before AChE injection and 5 min after. Mice were challenged with paraoxon (750 µg/kg) at 10 min post-injection and symptoms were observed. No supportive measures were given, and mice that did not show improvement within 15 min following challenge were euthanized. Experiments were approved by the institutional animal care and use committees of Arizona State University and The Hebrew University of Jerusalem.

***Biochemical and molecular analyses:*** Total genomic leaf DNA blot analysis was done a DIG-labeled probe (PCR-amplified with the primers ^{5'}GTCTGCTACCAATATGTGGACACCC^{3'} (SEQ ID NO: 13) and ^{5'}CCTGGAGGACAGCCCACAAGGTGGG^{3'} SEQ ID NO: 14) as previously described (Mor et al, 2001, Biotechnol. Bioeng. 75, 259-266). Purification of plant-derived AChE-R_{ER} from leaf tissue was as described (Geyer et al, 2005, Chem Biol Interact 157-158, 331-334). Typical preparations of pure enzyme had specific activities of ∼3,000 U/mg protein, and only preparations with specific activities larger than 2000 U/mg protein were used *in vivo.*

Protein preparations were resolved by SDS-PAGE, and were either stained with GelCode SilverSNAP Stain Kit II (Pierce), Coomassie brilliant blue, or transferred to a PVDF membrane and immunodecorated with polyclonal Abs raised either against the common domain unique to human or mouse AChE (N-19 and E-19 respectively, Santa Cruz) or the C-terminal domain of human AChE-R (Sternfeld et al., 2000, Proc Natl Acad Sci U S A 97, 8647-8652). HRP-conjugated secondary Abs (Sigma) and the ECL-plus kit (Amersham) were used for detection. Total soluble protein (TSP) was determined as described (Mor et al, 2001, Biotechnol. Bioeng. 75, 259-266).

AChE activity assays, enzyme kinetics, inhibition curves and AChE activity staining of non-denaturing PAGE were as previously described (Mor et al, 2001, Biotechnol. Bioeng. 75, 259-266). Activity assays and staining were performed in the presence of 0.5x10⁻⁵ M of the specific BChE inhibitor tetraisopropylpyrophosphoramide (iso-OMPA, Sigma).

For histochemical AChE activity staining of diaphragms, sacrificed mice were dissected to expose the diaphragm muscles, which were fixed *in situ* with 4 % paraformaldehyde for 5 min, dissected out and kept overnight in the fixative at room temperature. Following two washes with PBS, the muscles were incubated in Karnovsky's staining solution at 4 °C until brown precipitants appeared. NMJ analyses involved the Image-Pro Plus software (Media Cybernetids, Silver Spring, MD).

### EXAMPLE 5

### Production of human ER-trapped AChE-R in plants

Humans and other mammals express several molecular variants of AChE. Aii share a common core domain and enzymatic properties but possess distinct C-terminal peptides, expression patterns, subcellular localization and protein partner interactions (Figure 6A). Plants, like other heterologous expression systems, may produce recombinant human proteins that are decorated with non-human complex glycans, predicting their fast circulatory clearance. In addition, plant glycans may contain unique glyco-epitopes that may be allergenic in some cases. The present inventors, therefore, engineered AChE-R to contain the C-terminal peptide SEKDEL (Figure 6B), allowing its retrogade transport from the *cis*-Golgi back to the ER. These modifications enabled the present inventors to select a plant line, harboring at least four copies of the transgene (Figure 6C), that accumulated AChE-R_{ER} to 0.5 %-1.0 % TSP (∼19 U/mg protein, or ∼30 mg/kg fresh weight).

### RESULTS

The selected line was propagated for seed and biomass production in a USDA-approved containment greenhouse (Figure 6D). The plants appeared phenotypically indistinguishable from WT plants under normal growth conditions.

The AChE-R_{ER} protein could be detected in crude plant extracts by SDS PAGE, revealing a 67 kDa band absent from the WT's extract (Figure 7A). AChE-R_{ER} was purified to homogeneity from plant extracts using procainamide-affinity chromatography followed by anion-exchange chromatography (Geyer et al, 2005, Chem Biol Interact 157-158, 331-334) with final specific activity of the purified protein > 3000 U/mg. When resolved by SDS-PAGE and visualized either by Coomassie or silver staining, the purified protein appeared as a doublet that strongly reacts with AChE-specific Abs (Figures 7A,B). As predicted by its retention in the ER, the protein contains high-mannose glycans (data not shown) and the two bands apparent in the gel possibly represent two differently glycosylated species. Protein sequencing revealed that the accumulating enzyme was correctly processed with the mature protein's N-terminal residue being R35, as previously demonstrated for AChE expressed in a human cell line. Non-denaturing PAGE (Figure 7C) demonstrated that unlike recombinant AChE-S, ACnE-R_{ER} is monomeric, compatible with previous observations. Pure preparations of plant-produced AChE-R_{ER} were found to be stable at 4 °C for at least 12 months.

### EXAMPLE 6

### Kinetic characterization of plant-derived AChE-R_{ER}

Activities of plant-derived human AChE-R_{ER} and human AChE-S were compared to cell culture-derived human AChE-S (Soreq et al., 1990, Proc Natl Acad Sci U S A 87, 9688-9692, from Sigma). *K*ₘ values for all three variants were very similar to each other and to those reported in the literature (-0.2 mM, Figure 7D, Kronman et al. 1992, Gene 121, 295-304, Sternfeld et al., 1998, J. Neurosci. 18, 1240-1249). The enzymes exhibited characteristic inhibition by substrate conc. >2 mM, with no significant differences between the plant- and cell-derived AChE-S. Interestingly, substrate inhibition was more pronounced in the case of the R variant, compatible with C-terminus- active site intramolecular signaling. Only slight differences were observed in the ability of AChE-R_{ER} to bind and be inhibited by paraoxon, the active OP metabolite of the pesticide parathion (Figure 7E).

### EXAMPLE 7

### Circulatory clearance of plant-derived AChE-R_{ER}

The clearance of plant-derived AChE-R_{ER} in mice was determined by i.v. injection of enzyme (either 400 U or 1000 U) and measuring its residual activity in plasma samples in the presence of iso-OMPA (Figure 8A).

### RESULTS

The plant-derived human enzyme was cleared rapidly with circulatory *t*_{½} = 24 min, similar to observations with AChE from other sources (Kronman, C.,et al., (2005) Chem Biol Interact 157-158, 51-55).

Human AChE was detected by immunoblotting with Abs specific to the common domain of human AChE in plasma samples from mice treated with 400 U AChE-R_{ER} but not from control animals. AChE protein levels decreased after their initial peak at 10 min and were no longer detectable at 2 hr, compatible with the enzymatic assay (Figure 8A).

Non-denaturing PAGE revealed siow-migrating ChE bands, likely representing BChE oligomers inaccessible to iso-OMPA inhibition, in the plasma of all mice, including controls. Catalytically active monomeric enzyme was, however, observed in plasma of AChE-R_{ER}-injected, but not PBS-injected controls (Figure 8C). Surprisingly, compared to the purified plant-derived AChE-R_{ER} (Figure 8C, left lane), the injected enzyme in the mouse plasma samples migrated more slowly as several broad bands (Figure 8C).

### EXAMPLE 8

### Protection from OP challenge by plant-produced AChE-R_{ER}

To test if exogenously applied AChE-R_{ER} could protect animals from an OP challenge, the present inventors determined the effect of an experimental single-bolus LD₅₀ of paraoxon.

### RESULTS

In 6-8 week old male FVB/N mice, the LD₅₀ was 660 µg/kg. Pretreatment with increasing amounts of plant-produced human AChE-R_{ER} (dose adjusted to body weight) was then followed by challenge with 1.14 x LD₅₀ paraoxon and symptoms were observed and recorded (Figures 9A-B). This dose of paraoxon is 100 % fatal for otherwise untreated mice, with death preceded by severe cholinergic symptoms (convulsions and strained respiratory ability), occurring within 5-10 min. Pretreating 17 mice with plant-derived AChE-R_{ER} reduced mortality even when the molar ratio of AChE-R_{ER}/OP was as low as 0.04, far lower than the comparable stoichiometric protection by BChE. At this low molar ratio, the surviving animals initially displayed severe symptoms followed by more moderate symptoms (less drastic convulsions and tremor). Symptoms gradually diminished and within 4 hours, mice exhibited only mild indications (decreased motor activity and general malaise). Importantly, all mice that survived the first 15 min following paraoxon injection showed apparently complete recovery within 24 hours.

Larger prophylactic doses of plant-derived AChE-R_{ER} provided yet better protection. At enzyme/agent molar ratios >0.09, initial symptoms were moderate, and at >0.2 the initial symptoms were only mild. Mice treated with enzyme/agent ratio of >0.5 did not show any signs of post-exposure symptoms. Six-month monitoring of all surviving mice revealed no obvious neuropathy or other symptoms. Exceedingly low titers of IgGs specific to the administered human enzyme were observed; however neither AChE-specific IgEs nor auto-Abs directed against murine AChE could be detected in serum samples from mice treated even with the largest dose of plant-derived AChE-R_{ER} (∼10,00 U, data not shown).

### EXAMPLE 9

### AChE-R_{ER} treatment limits the OP-induced upreculation of host AChE in plasma

OP exposure induces two seemingly inverse effects: First, it decreases ChE activities by blockade, and second, it induces long-lasting feedback overproduction of AChE-R. In another set of experiments, the present inventors examined the ability of AChE-R_{ER} to protect from these delayed effects of OP poisoning. To this end, mice were pre-treated by i.v. administration of PBS +/- AChE-R_{ER} (400 U) followed by a sublethal (0.8 LD₅₀) or lethal (1.13 LD₅₀) paraoxon challenge.

### RESULTS

Unprotected, sub-lethally-challenged mice, or AChE-protected, lethally-challenged mice all exhibited severe cholinergic symptoms followed by recovery (survival rates were 100 % and 70 % for sub-lethally- and lethally-challenged mice, respectively). Ten days post-treatment, mice were euthanized and plasma and skeletal muscle samples were harvested. At this time, all mice predictably experienced a small increase in their plasma AChE levels induced by the OP challenge (Figure 9B, Figure 10A). Plasma AChE activity at this delayed time point was substantially lower in enzyme-treated animals as compared to unprotected sub-lethally challenged mice, suggesting an effective yet incomplete offset of the long-lasting AChE-R over-production by administration of plant-derived AChE-R_{ER}. Administration of plant-derived AChE-R_{ER} alone significantly limited the levels of plasma AChE-R activity 10 days post injection (Figure 9B, Figure 10A), supporting the notion that this offset was a feedback response to the high levels of AChE molecules in the circulatory system. Furthermore, the enzyme-pretreatment modulation of the OP-induced increase in plasma AChE activity is mirrored by corresponding changes in AChE monomers, probably murine AChE-R, observed by non-denaturing gels of samples obtained 10 days post-challenge (Figure 10A). It therefore may be concluded that the post-poisoning increase in the plasma levels of endogenous murine ACHE. specifically murine AChE-R, can be at least partially mitigated by exogenously applied plant-derived enzyme.

### EXAMPLE 10

### AChE-R_{ER} treatment ameliorates NMJ dismorphology induced by OP poisoning.

### RESULTS

Enzyme pretreatment exerted considerable attenuation effects on the post-exposure accumulation of the murine AChE-R in skeletal (leg) muscles following both lethal and sublethal OP insults and prevented its inhibition (Figure 10C). At the same time, muscle AChE activities in AChE-R_{ER} pre-treated mice were similar to those of unchallenged controls (Figures 10D-E) and compatible with the hypothesis that the administered AChE-R_{ER} served as a "decoy" protecting muscle AChE-R from inhibition. It therefore appears that administration of plant-derived AChE-R_{ER} reciprocally modulated the OP-induced changes in AChE gene expression, preventing its inhibition in muscles while decreasing OP-induced AChE-R production in the mouse circulation.

To study the effect of AChE-R_{ER} on the long-term dismorphology of NMJs induced by OPs, intact diaphragm muscles were dissected 10 days post-treatment, stained for catalytically active AChE and analyzed with respect to NMJ density and mean individual area (Figures 11A-B). Paraoxon exposure increased both the density and the fraction of large NMJs (Figures 11A-B indicates *P*<0.01, median test) as compared to the PBS control. Pre-treatment with 400U AChE-R_{ER} prevented at least part of the exposure-induced enlargement of NMJ area, whereas treatment with 400U AChE-R_{ER} alone did not alter NMJ density or area within the non-exposed diaphragm (P> 0.05, median test, Figures 11A-B). These findings attribute the attenuated NMJ dismorphology effects to ameliorated feedback response in host muscle tissues.

### EXAMPLE 11

### Ability of AChE-R_{ER} to protect from these delayed effects of OP poisoning

OP exposure induces two seemingly inverse effects: First, it decreases ChE activities by blockade, and second, it induces long-lasting feedback overproduction of AChE-R. In this example, the present inventors examined the ability of AChE-R_{ER} to protect from these delayed effects of OP poisoning. To this end, mice were pre-treated by i.v. administration of PBS +/- AChE-R_{ER} (400 U) followed by a sublethal (0.8 LD₅₀) or lethal (1.13 LD₅₀) paraoxon challenge.

### RESULTS

Unprotected, sub-lethally-challenged mice, or AChE-protected, lethally-challenged mice all exhibited severe cholinergic symptoms followed by recovery (survival rates were 100 % and 70 % for sub-lethally- and lethally-challenged mice, respectively). Ten days post-treatment, mice were euthanized and plasma and skeletal muscle samples were harvested. At this time, all mice predictably experienced a small increase in their plasma AChE levels induced by the OP challenge (Figure 9B, Figure 10A). Plasma AChE activity at this delayed time point was substantially lower in enzyme-treated animals as compared to unprotected sub-lethally challenged mice, suggesting an effective yet incomplete offset of the long-lasting AChE-R over-production by administration of plant-derived AChE-R_{ER}. Administration of plant-derived AChE-R_{ER} alone significantly limited the levels of plasma AChE-R activity 10 days post injection (Figure 9B, Figure 10A), supporting the notion that this offset was a feedback response to the high levels of AChE molecules in the circulatory system. Furthermore, the enzyme-pretreatment modulation of the OP-induced increase in plasma AChE activity is mirrored by corresponding changes in AChE monomers, probably murine AChE-R, observed by non-denaturing gels of samples obtained 10 days post-challenge (Figure 10A).

The enzyme-pretreatment modulation of the OP-induced increase in plasma AChE activity is mirrored by corresponding changes in AChE monomers, probably murine AChE-R, observed by non-denaturing gels of samples obtained 10 days post-challenge (Figure 10A). These monomers migrate somewhat slower than the plant-derived pure AChE-R_{ER}, which were completely cleared by the mice 2 hours after their administration (Figure 8A-C). The slower migration species can either reflect protein-protein associations or distinct glycosylation patterns of post-challenge murine AChE-R.

To detect long-term changes in the total amount of AChE (active and inactive), plasma proteins were resolved by SDS-PAGE followed by detection of the AChE protein using specific Abs directed against the AChE common domain or against the unique carboxyl-terminal domain of murine AChE-R (Figure 10B). Individual mice in each treatment group presented considerable variability. Nevertheless, paraoxon-induced, dose-dependent elevation of murine AChE in the blood appeared to be countered by the enzyme pretreatment. Thus, plasma accumulation of murine AChE-R paralleled the trend of changes in total AChE (Figure 10B) and matched the trends seen with AChE activity and active AChE monomers (Figure 9B, 10A). It therefore may be concluded that the post-poisoning increase in the plasma levels of endogenous murine AChE, specifically murine AChE-R, can be at least partially mitigated by exogenously applied plant-derived enzyme.

Enzyme pretreatment exerted considerable attenuation effects on the post-exposure accumulation of the murine AChE-R in skeletal (leg) muscles following both lethal and sublethal OP insults and prevented its inhibition (Figure 10C).

In muscles, the vast majority of AChE molecules, even following insult, is of the synaptic variant, AChE-S. AChE-S can be further organized into several molecular forms, including soluble and membrane bound ones, which can be separated by sequential extraction of tissue homogenates. Homogenization in low-salt buffer releases soluble (monomeric) AChE-S and AChE-R (together representing ∼15 % of total AChE activity, Figure 10E). Subsequent extractions, first with buffer containing 1 % Triton X-100 (Low Salt-Detergent, ∼65 %), then with 1 M NaCl (High Salt, ∼20 %) release the AChE-S species, mostly as tetramers that are associated with membranes (data not shown). Administration of plant-derived AChE-R_{ER} had no significant effect on the total muscle AChE activity, but a lethal dose of paraoxon caused a ∼50 % decrease in membrane-bound detergent-soluble forms of AChE (Figure 10E), most probably tetrameric AChE-S. In unprotected mice, paraoxon also caused a significant decrease in the low-salt soluble AChE activity (presumably AChE-R) (-16.4 %, Figure 10E), suggesting that host muscle AChE-R is irreversibly inhibited by the OP, yielding "aged" enzyme. AChE-R_{ER} treatment reversed this trend, so that AChE activities were apparently similar to those of PBS controls (Figure 10E) and compatible with the hypothesis that the administered AChE-R_{ER} served as a "decoy" protecting muscle AChE-R from inhibition. In conclusion, administration of plant-derived AChE-R_{ER} reciprocally modulated the OP-induced changes in AChE gene expression, preventing its inhibition in muscles while decreasing OP-induced AChE-R production in the mouse circulation.

### DISCUSSION

The search for a useful prophylactic and/or therapeutic agent to counteract the effects of OP poisoning must consider many factors, including production costs, safety and, of course, the ability to ensure both short and long-term protection. Here the present inventors have demonstrated that plant production addresses the first two concerns while choosing the soluble stress-related "readthrough" splice variant of AChE addresses the third and fourth.

The present inventors expressed human AChE-R_{ER} in transgenic plants, which have recently emerged as a promising system, still in its experimental infancy, for the production of protein-pharmaceuticals. Plant systems offer low production costs (with comparable purification and regulatory costs), production scalability and flexibility (with low capital investment), and improved safety (no concern of human pathogens and prions). Similar to other heterologous expression systems, the N-linked glycosylation process in plants differs in details from that in humans. In this context, two major concerns are raised: accelerated circulatory clearance because of the inability of plants to sialilate their glycoproteins and risk of increased allergenicity because of the presence of unique plant glyco-epitopes on complex glycans. To minimize these risks, AChE-R accumulation was directed to the ER, thus avoiding the *trans-Golgi,* where xylose and fucose residues, occasionally associated with allergic reactions in humans, are added. Indeed, preliminary data show that plant-derived AChE-R_{ER} is decorated by high-mannose glycans, which are not different from those of humans and therefore lacking the "allergenic" glyco-epitopes.

ER retention of the recombinant AChE-R, allowed its accumulation to very high levels (∼1% TSP). Similar to its native counterpart, the plant-derived protein was cleared rapidly from the circulation. The circulatory *t*_{½} of mammalian ChEs depends on amino acid domains on the protein's surface, on glycan structures and on the protein's oligomerization state. Elimination rate is influenced by the species in which it is measured, by the genomic source of the tested protein, and by the production host. For example, clearance in primates of simian and human AChE-S (produced in a human cell line) is only marginally affected by the protein's oligomerization state or its glycosylation. Similarly, BChE purified from human blood showed characteristically long circulatory *t*_{½} in rodents, while recombinant human BChE produced in a hamster cell line or in the mammary glands of transgenic goats was eliminated much faster. The inferior pharmacokinetic parameters encountered by administration of recombinant ChEs can be improved by decorating the proteins with poly (ethylene glycol), which masks the surface features responsible for the rapid clearance. Accordingly, "PEGytated" recombinant human AChE-S or recombinant BChE derived from milk of transgenic goats displayed significantly longer circulatory *t*_{½} as compared to the non-PEGylated proteins in either rodents, pigs or monkeys.

The examples herein above demonstrate that recombinant human AChE-R_{ER}, can accumulate in transgenic plant tissues to commercially-viable levels, that it can be readily purified to homogeneity, and that it possesses the kinetic hallmarks of the authentic human enzyme in respect to substrate hydrolysis and OP binding. Plant production of ER-trapped, soluble AChE-R was found to be advantageous at several levels. Recombinant human AChE-R_{ER} accumulates in plant tissues to commercially-viable levels (Figures 6A-D). When purified to homogeneity, it reaches the kinetic hallmarks of the authentic human enzyme with respect to substrate hydrolysis and OP binding (Figures 7A-E). Its near stoichiometric administration to animals dramatically raised the level of circulating ChEs (Figures 8A-C), fully protecting them from the acute symptoms of OP poisoning and death (Figures 9A-B). Furthermore, AChE-R prophylaxis ameliorated the chronic effects of OP toxicity by mitigating the long-term upregulation of *ACHE* gene expression (Figures 9A-B and Figures 10A-E), adding a new dimension to the concept of ChE therapy. Over and above its function as a passive OP-bioscavenger, this particular isoform of AChE can actively interfere with the vicious cycle associated with the long-lasting molecular feedback response to OP exposure.

Exposure to OP ChE inhibitors rapidly increases the levels of synaptic ACh in NMJs and in peripheral tissues, initiating the so-called "cholinergic crisis" (Figure 11C). AChE-R overproduction quickly restores the cholinergic balance by increasing the ACh hydrolysis potential throughout the circulation; however, persistent overproduction of AChE-R associates with overproduction of pro-inflammatory cytokines, behavioral impairments, declarative memory loss, muscle malfunctioning, and excessive myelopoiesis. Pretreatment with the enzyme would likely cause a transient elevation of inflammation markers; however, the built-in transience of the plant-derived AChE-R_{ER} would limit the duration of such effects. Moreover, mitigated overproduction of endogenous AChE-R in the circulation should further limit the duration of such symptoms, suggesting that pre-treatment with this particular AChE isoform can not only prevent the acute cholinergic crisis following OP intoxication but may also limit its deleterious chronic aftermath.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. Use of AChE-R for the treating or preventing organophosphate exposure associated damage, wherein said AChE-R is (a) recombinant or (b) plant produced or both; and (c) as set forth in SEQ ID NO: 4.

2. The use of claim 1, wherein said AChE-R is formulated for inhalation.

3. The use of claim 1, wherein said AChE-R is formulated for injection.

4. The use of claim 1, wherein said AChE-R is formulated for topical application.

5. An article of manufacture for treating or preventing organophosphate exposure associated damage, the article of manufacture comprising AChE-R immobilized onto a solid support, wherein said AChE-R is (a) recombinant or (b) plant produced or both; and (c) as set forth in SEQ ID NO: 4.

6. The article of manufacture of claim 5, wherein said solid support is for topical administration.

7. The article of manufacture of claim 5, wherein said solid support is selected from the group consisting of a filter, a fabric and a lining.

8. A method of detoxifying a surface, the method comprising contacting the surface with AChE-R, wherein said AChE-R is (a) recombinant or (b) plant produced or both; and (c) as set forth in SEQ ID NO: 4, thereby detoxifying the surface.

9. A coating material comprising AChE-R, wherein said AChE-R is (a) recombinant or (b) plant produced or both; and (c) as set forth in SEQ ID NO: 4.


SEQUENCE LISTING

<110>  Yissum Research Development Company of the Hebrew
       University of Jerusalem

<120>  MATERIALS AND METHODS FOR TREATING OR PREVENTING ORGANOPHOSPHATE
       EXPOSURE ASSOCIATED DAMAGE

<130>  F-21035/EP-SSP

<140>  07805492.1
<141>  20.08.2007

<160>  15

<170>  PatentIn version 3.4

<210>  1
<211>  1806
<212>  DNA
<213>  Artificial sequence

<220>
<223>  Acetylcholinesterase readthrough isoform (oAChE-R) synthetic
       construct

<400>  1
atgggaaggc ctccacaatg tctcctccac actccttcct tggcttcccc actccttctc      60

ctcctcctct ggctcttggg tggaggagtg ggtgctgagg gtagggagga tgctgaactc     120

ttggtgactg tgagaggagg aaggctcagg ggcattagac tcaagacccc aggtggccct     180

gtctctgctt tcttgggcat cccctttgct gagccaccaa tgggacccag aaggtttctc     240

ccacctgagc ccaagcaacc ttggtcagga gtggttgatg ctacaacctt ccaatctgtc     300

tgctaccaat atgtggacac cctttaccca ggttttgagg gcactgagat gtggaacccc     360

aacagagaac tctctgagga ctgcctctac ctcaatgtgt ggacaccata ccccaggcct     420

acatctccca cccctgtctt ggtctggatc tatggtggtg gcttctactc aggagcctcc     480

tccttggatg tgtatgatgg aagattcttg gttcaagctg agaggactgt gttggtgtcc     540

atgaactaca gggtgggagc ctttggcttc ttggccctcc caggttccag agaggcccct     600

ggcaatgtgg gtctcttgga tcaaaggttg gccctccaat gggtgcaaga gaatgtggct     660

gcctttggag gtgacccaac atcagtgact ctctttggtg agtctgctgg agctgcctca     720

gtgggcatgc acctcctctc ccctccctcc aggggcctct tccacagggc tgtgctccaa     780

tctggcgcgc ccaatggtcc ctgggccact gtgggcatgg gagaggccag aaggagggcc     840

actcagttgg cccaccttgt gggctgtcct ccaggaggca ctggtggaaa tgacacagag     900

ttggttgcct gccttaggac aagaccagca caagtcttgg tgaaccatga atggcatgtg     960

ctccctcaag aatctgtctt caggttctcc tttgtgcctg tggttgatgg agacttcctc    1020

tctgacaccc cagaggccct catcaatgct ggagactttc atggcctcca agtgttggtg    1080

ggtgtggtga aggatgaggg ctcatatttc ttggtttatg gtgccccagg cttcagcaag    1140

gacaatgagt ctctcatctc cagggctgag ttcttggctg gagtgagggt gggtgttccc 1200

caagtttcag acttggctgc tgaggctgtg gtcctccatt acacagactg gctccatcca 1260

gaggacccag caagactcag ggaggccctc tctgatgtgg tgggagacca caatgtggtg 1320

tgcccagtgg cacaattggc tggaagattg gctgcccaag gtgccagggt ctatgcctat 1380

gtctttgaac acagagcttc cactctctcc tggcccctct ggatgggtgt gcctcatggc 1440

tatgagattg agttcatctt tggaatcccc ttggacccct ctagaaacta cactgctgag 1500

gagaagatct ttgcccaaag actcatgaga tactgggcca actttgccag gacaggtgat 1560

cccaatgagc ccagagaccc caaggcccca caatggccac cttacactgc tggagcacaa 1620

caatatgttt ctttggacct caggccattg gaggtgagga ggggtctcag agcccaagcc 1680

tgtgccttct ggaacaggtt cctccccaag ttgctctctg ccaccggtat gcaaggacca 1740

gctggctctg gctgggagga gggtagtggt agcccaccag gtgtaacccc tctcttctct 1800

ccctag 1806

<210> 2
<211> 601
<212> PRT
<213> Artificial sequence

<220>
<223> Acetylcholinesterase readthrough isoform (oAChE-R) polypeptide
sequence

<400> 2

Met Gly Arg Pro Pro Gln Cys Leu Leu His Thr Pro Ser Leu Ala Ser
1               5                   10                  15

Pro Leu Leu Leu Leu Leu Leu Trp Leu Leu Gly Gly Gly Val Gly Ala
            20                  25                  30

Glu Gly Arg Glu Asp Ala Glu Leu Leu Val Thr Val Arg Gly Gly Arg
        35                  40                  45

Leu Arg Gly Ile Arg Leu Lys Thr Pro Gly Gly Pro Val Ser Ala Phe
    50                  55                  60

Leu Gly Ile Pro Phe Ala Glu Pro Pro Met Gly Pro Arg Arg Phe Leu
65                  70                  75                  80

Pro Pro Glu Pro Lys Gln Pro Trp Ser Gly Val Val Asp Ala Thr Thr
                85                  90                  95

Phe Gln Ser Val Cys Tyr Gln Tyr Val Asp Thr Leu Tyr Pro Gly Phe
            100                 105                 110

Glu Gly Thr Glu Met Trp Asn Pro Asn Arg Glu Leu Ser Glu Asp Cys
        115                 120                 125

Leu Tyr Leu Asn Val Trp Thr Pro Tyr Pro Arg Pro Thr Ser Pro Thr
    130                 135                 140

Pro Val Leu Val Trp Ile Tyr Gly Gly Gly Phe Tyr Ser Gly Ala Ser
145                 150                 155                 160

Ser Leu Asp Val Tyr Asp Gly Arg Phe Leu Val Gln Ala Glu Arg Thr
                165                 170                 175

Val Leu Val Ser Met Asn Tyr Arg Val Gly Ala Phe Gly Phe Leu Ala
            180                 185                 190

Leu Pro Gly Ser Arg Glu Ala Pro Gly Asn Val Gly Leu Leu Asp Gln
        195                 200                 205

Arg Leu Ala Leu Gln Trp Val Gln Glu Asn Val Ala Ala Phe Gly Gly
    210                 215                 220

Asp Pro Thr Ser Val Thr Leu Phe Gly Glu Ser Ala Gly Ala Ala Ser
225                 230                 235                 240

Val Gly Met His Leu Leu Ser Pro Pro Ser Arg Gly Leu Phe His Arg
                245                 250                 255

Ala Val Leu Gln Ser Gly Ala Pro Asn Gly Pro Trp Ala Thr Val Gly
            260                 265                 270

Met Gly Glu Ala Arg Arg Arg Ala Thr Gln Leu Ala His Leu Val Gly
        275                 280                 285

Cys Pro Pro Gly Gly Thr Gly Gly Asn Asp Thr Glu Leu Val Ala Cys
    290                 295                 300

Leu Arg Thr Arg Pro Ala Gln Val Leu Val Asn His Glu Trp His Val
305                 310                 315                 320

Leu Pro Gln Glu Ser Val Phe Arg Phe Ser Phe Val Pro Val Val Asp
                325                 330                 335

Gly Asp Phe Leu Ser Asp Thr Pro Glu Ala Leu Ile Asn Ala Gly Asp
            340                 345                 350

Phe His Gly Leu Gln Val Leu Val Gly Val Val Lys Asp Glu Gly Ser
        355                 360                 365

Tyr Phe Leu Val Tyr Gly Ala Pro Gly Phe Ser Lys Asp Asn Glu Ser

370                 375                 380

Leu Ile Ser Arg Ala Glu Phe Leu Ala Gly Val Arg Val Gly Val Pro
385                 390                 395                 400

Gln Val Ser Asp Leu Ala Ala Glu Ala Val Val Leu His Tyr Thr Asp
                405                 410                 415

Trp Leu His Pro Glu Asp Pro Ala Arg Leu Arg Glu Ala Leu Ser Asp
            420                 425                 430

Val Val Gly Asp His Asn Val Val Cys Pro Val Ala Gln Leu Ala Gly
        435                 440                 445

Arg Leu Ala Ala Gln Gly Ala Arg Val Tyr Ala Tyr Val Phe Glu His
    450                 455                 460

Arg Ala Ser Thr Leu Ser Trp Pro Leu Trp Met Gly Val Pro His Gly
465                 470                 475                 480

Tyr Glu Ile Glu Phe Ile Phe Gly Ile Pro Leu Asp Pro Ser Arg Asn
                485                 490                 495

Tyr Thr Ala Glu Glu Lys Ile Phe Ala Gln Arg Leu Met Arg Tyr Trp
            500                 505                 510

Ala Asn Phe Ala Arg Thr Gly Asp Pro Asn Glu Pro Arg Asp Pro Lys
        515                 520                 525

Ala Pro Gln Trp Pro Pro Tyr Thr Ala Gly Ala Gln Gln Tyr Val Ser
    530                 535                 540

Leu Asp Leu Arg Pro Leu Glu Val Arg Arg Gly Leu Arg Ala Gln Ala
545                 550                 555                 560

Cys Ala Phe Trp Asn Arg Phe Leu Pro Lys Leu Leu Ser Ala Thr Gly
                565                 570                 575

Met Gln Gly Pro Ala Gly Ser Gly Trp Glu Glu Gly Ser Gly Ser Pro
            580                 585                 590

Pro Gly Val Thr Pro Leu Phe Ser Pro
        595                 600

<210>  3
<211>  1821
<212>  DNA
<213>  Artificial sequence

<220>

<223>  Acetylcholinesterase fused to the C-terminal ER-retention signal
      (hAChE-R ER) polynucleotide sequence

<400>  3
atgaggcctc cacaatgtct cctccacact ccttccttgg cttccccact ccttctcctc      60

ctcctctggc tcttgggtgg aggagtgggt gctgagggta gggaggatgc tgaactcttg     120

gtgactgtga gaggaggaag gctcaggggc attagactca agaccccagg tggccctgtc     180

tctgctttct tgggcatccc ctttgctgag ccaccaatgg gacccagaag gtttctccca     240

cctgagccca agcaaccttg gtcaggagtg gttgatgcta caaccttcca atctgtctgc     300

taccaatatg tggacaccct ttacccaggt tttgagggca ctgagatgtg gaaccccaac     360

agagaactct ctgaggactg cctctacctc aatgtgtgga caccataccc caggcctaca     420

tctcccaccc ctgtcttggt ctggatctat ggtggtggct tctactcggg agcctcctcc     480

ttggatgtgt atgatggaag attcttggtt caagctgaga ggactgtgtt ggtgtccatg     540

aactacaggg tgggagcctt tggcttcttg gccctcccag gttccagaga ggcccctggc     600

aatgtgggtc tcttggatca aaggttggcc ctccaatggg tgcaagagaa tgtggctgcc     660

tttggaggtg acccaacatc agtgactctc tttggtgagt ctgctggagc tgcctcagtg     720

ggcatgcacc tcctctcccc tccctccagg ggcctcttcc acagggctgt gctccaatct     780

ggcgcgccca atggtccctg ggccactgtg ggcatgggag aggccagaag gagggccact     840

cagttggccc accttgtggg ctgtcctcca ggaggcactg gtggaaatga cacagagttg     900

gttgcctgcc ttaggacaag accagcacaa gtcttggtga accatgaatg gcatgtgctc     960

cctcaagaat ctgtcttcag gttctccttt gtgcctgtgg ttgatggaga cttcctctct    1020

gacaccccag aggccctcat caatgctgga gactttcatg gcctccaagt gttggtgggt    1080

gtggtgaagg atgagggctc atatttcttg gtttatggtg ccccaggctt cagcaaggac    1140

aatgagtctc tcatctccag ggctgagttc ttggctggag tgagggtggg tgttccccaa    1200

gtttcagact tggctgctga ggctgtggtc ctccattaca cagactggct ccatccagag    1260

gacccagcaa gactcaggga ggccctctct gatgtggtgg gagaccacaa tgtggtgtgc    1320

ccagtggcac aattggctgg aagattggct gcccaaggtg ccagggtcta tgcctatgtc    1380

tttgaacaca gagcttccac tctctcctgg cccctctgga tgggtgtgcc tcatggctat    1440

gagattgagt tcatctttgg aatccccttg gacccctcta gaaactacac tgctgaggag    1500

aagatctttg cccaaagact catgagatac tgggccaact ttgccaggac aggtgatccc    1560

aatgagccca gagaccccaa ggccccacaa tggccacctt acactgctgg agcacaacaa    1620

tatgtttctt tggacctcag gccattggag gtgaggaggg gtctcagagc ccaagcctgt    1680

gccttctgga acaggttcct ccccaagttg ctctctgcca ccggtatgca aggaccagct    1740

ggctctggct gggaggaggg tagtggtagc ccaccaggtg taacccctct cttctctccc    1800

tctgagaagg atgaactcta g                                              1821

<210>  4
<211>  606
<212>  PRT
<213>  Artificial sequence

<220>
<223>  Acetylcholinesterase fused to the C-terminal ER-retention signal
       (hAChE-R ER) polypeptide sequence

<400>  4

Met Arg Pro Pro Gln Cys Leu Leu His Thr Pro Ser Leu Ala Ser Pro
1               5                   10                  15

Leu Leu Leu Leu Leu Leu Trp Leu Leu Gly Gly Gly Val Gly Ala Glu
            20                  25                  30

Gly Arg Glu Asp Ala Glu Leu Leu Val Thr Val Arg Gly Gly Arg Leu
        35                  40                  45

Arg Gly Ile Arg Leu Lys Thr Pro Gly Gly Pro Val Ser Ala Phe Leu
    50                  55                  60

Gly Ile Pro Phe Ala Glu Pro Pro Met Gly Pro Arg Arg Phe Leu Pro
65                  70                  75                  80

Pro Glu Pro Lys Gln Pro Trp Ser Gly Val Val Asp Ala Thr Thr Phe
                85                  90                  95

Gln Ser Val Cys Tyr Gln Tyr Val Asp Thr Leu Tyr Pro Gly Phe Glu
            100                 105                 110

Gly Thr Glu Met Trp Asn Pro Asn Arg Glu Leu Ser Glu Asp Cys Leu
        115                 120                 125

Tyr Leu Asn Val Trp Thr Pro Tyr Pro Arg Pro Thr Ser Pro Thr Pro
    130                 135                 140

Val Leu Val Trp Ile Tyr Gly Gly Gly Phe Tyr Ser Gly Ala Ser Ser
145                 150                 155                 160

Leu Asp Val Tyr Asp Gly Arg Phe Leu Val Gln Ala Glu Arg Thr Val
                165                 170                 175

Leu Val Ser Met Asn Tyr Arg Val Gly Ala Phe Gly Phe Leu Ala Leu
            180                 185                 190

Pro Gly Ser Arg Glu Ala Pro Gly Asn Val Gly Leu Leu Asp Gln Arg
        195                 200                 205

Leu Ala Leu Gln Trp Val Gln Glu Asn Val Ala Ala Phe Gly Gly Asp
    210                 215                 220

Pro Thr Ser Val Thr Leu Phe Gly Glu Ser Ala Gly Ala Ala Ser Val
225                 230                 235                 240

Gly Met His Leu Leu Ser Pro Pro Ser Arg Gly Leu Phe His Arg Ala
                245                 250                 255

Val Leu Gln Ser Gly Ala Pro Asn Gly Pro Trp Ala Thr Val Gly Met
            260                 265                 270

Gly Glu Ala Arg Arg Arg Ala Thr Gln Leu Ala His Leu Val Gly Cys
        275                 280                 285

Pro Pro Gly Gly Thr Gly Gly Asn Asp Thr Glu Leu Val Ala Cys Leu
    290                 295                 300

Arg Thr Arg Pro Ala Gln Val Leu Val Asn His Glu Trp His Val Leu
305                 310                 315                 320

Pro Gln Glu Ser Val Phe Arg Phe Ser Phe Val Pro Val Val Asp Gly
                325                 330                 335

Asp Phe Leu Ser Asp Thr Pro Glu Ala Leu Ile Asn Ala Gly Asp Phe
            340                 345                 350

His Gly Leu Gln Val Leu Val Gly Val Val Lys Asp Glu Gly Ser Tyr
        355                 360                 365

Phe Leu Val Tyr Gly Ala Pro Gly Phe Ser Lys Asp Asn Glu Ser Leu
    370                 375                 380

Ile Ser Arg Ala Glu Phe Leu Ala Gly Val Arg Val Gly Val Pro Gln
385                 390                 395                 400

Val Ser Asp Leu Ala Ala Glu Ala Val Val Leu His Tyr Thr Asp Trp
                405                 410                 415

Leu His Pro Glu Asp Pro Ala Arg Leu Arg Glu Ala Leu Ser Asp Val
            420                 425                 430

Val Gly Asp His Asn Val Val Cys Pro Val Ala Gln Leu Ala Gly Arg
        435                 440                 445

Leu Ala Ala Gln Gly Ala Arg Val Tyr Ala Tyr Val Phe Glu His Arg
    450                 455                 460

Ala Ser Thr Leu Ser Trp Pro Leu Trp Met Gly Val Pro His Gly Tyr

465                 470                 475                 480

Glu Ile Glu Phe Ile Phe Gly Ile Pro Leu Asp Pro Ser Arg Asn Tyr
                485                 490                 495

Thr Ala Glu Glu Lys Ile Phe Ala Gln Arg Leu Met Arg Tyr Trp Ala
            500                 505                 510

Asn Phe Ala Arg Thr Gly Asp Pro Asn Glu Pro Arg Asp Pro Lys Ala
        515                 520                 525

Pro Gln Trp Pro Pro Tyr Thr Ala Gly Ala Gln Gln Tyr Val Ser Leu
    530                 535                 540

Asp Leu Arg Pro Leu Glu Val Arg Arg Gly Leu Arg Ala Gln Ala Cys
545                 550                 555                 560

Ala Phe Trp Asn Arg Phe Leu Pro Lys Leu Leu Ser Ala Thr Gly Met
                565                 570                 575

Gln Gly Pro Ala Gly Ser Gly Trp Glu Glu Gly Ser Gly Ser Pro Pro
            580                 585                 590

Gly Val Thr Pro Leu Phe Ser Pro Ser Glu Lys Asp Glu Leu
        595                 600                 605

<210>  5
<211>  80
<212>  DNA
<213>  Artificial sequence

<220>
<223>  A polynucleotide expressing hARP - an active portion of AChE-R

<400>  5
gtatgcaggg gccagcgggc agcggctggg aggaggggag tgggagcccg ccaggtgtaa      60

cccctctctt ctccccctag                                                  80

<210>  6
<211>  26
<212>  PRT
<213>  Artificial sequence

<220>
<223>  hARP - an active portion of AChE-R

<400>  6

Gly Met Gln Gly Pro Ala Gly Ser Gly Trp Glu Glu Gly Ser Gly Ser
1               5                   10                  15

Pro Pro Gly Val Thr Pro Leu Phe Ser Pro
            20                  25

<210>  7
<211>  116
<212>  DNA
<213>  Artificial sequence

<220>
<223>  A polynucleotide expressing mARP - an active portion of AChE-R

<220>
<221>  misc_feature
<222>  (90)..(92)
<223>  TAA is a stop codon within the intron

<400>  7
gtaggcgcat ggagtggggg gagcagggaa tgcacaaggc ggcaagggtt ggaaggaggg     60

gagagagatg gggtgcaaaa cacagggtgt aatccctctc ttctccacct ccccag       116

<210>  8
<211>  30
<212>  PRT
<213>  Artificial sequence

<220>
<223>  mARP - an active portion of AChE-R

<400>  8

Gly Arg Arg Met Glu Trp Gly Glu Gln Gly Met His Lys Ala Ala Arg
1               5                   10                  15

Val Gly Arg Arg Gly Glu Arg Trp Gly Ala Lys His Arg Val
            20                  25                  30

<210>  9
<211>  16
<212>  DNA
<213>  Artificial sequence

<220>
<223>  Single strand DNA oligonucleotide

<400>  9
ggtagacggg gacttc                                                    16

<210>  10
<211>  17
<212>  DNA
<213>  Artificial sequence

<220>
<223>  Single strand DNA oligonucleotide

<400>  10
atgtaggcat agacccg                                                   17

<210>  11

<211>  18
<212>  DNA
<213>  Artificial sequence

<220>
<223>  Single strand DNA oligonucleotide

<400>  11
cgggtctatg cctacatc                                                    18


<210>  12
<211>  21
<212>  DNA
<213>  Artificial sequence

<220>
<223>  Single strand DNA oligonucleotide

<400>  12
gctcggtcgt attatatccc a                                                21


<210>  13
<211>  25
<212>  DNA
<213>  Artificial sequence

<220>
<223>  Single strand DNA oligonucleotide

<400>  13
gtctgctacc aatatgtgga caccc                                            25


<210>  14
<211>  25
<212>  DNA
<213>  Artificial sequence

<220>
<223>  Single strand DNA oligonucleotide

<400>  14
cctggaggac agcccacaag gtggg                                            25


<210>  15
<211>  2156
<212>  DNA
<213>  Homo sapiens

<400>  15
cagcctgcgc cggggaacat cggccgcctc cagctcccgg cgcggcccgg cccggcccgg      60

ctcggccgcc tcagacgccg cctgccctgc agccatgagg cccccgcagt gtctgctgca     120

cacgccttcc ctggcttccc cactccttct cctcctcctc tggctcctgg gtggaggagt     180

gggggctgag ggccgggagg atgcagagct gctggtgacg gtgcgtgggg gccggctgcg     240

gggcattcgc ctgaagaccc ccgggggccc tgtctctgct ttcctgggca tcccctttgc     300

ggagccaccc atgggacccc gtcgctttct gccaccggag cccaagcagc cttggtcagg     360

ggtggtagac gctacaacct tccagagtgt ctgctaccaa tatgtggaca ccctataccc 420

aggttttgag ggcaccgaga tgtggaaccc caaccgtgag ctgagcgagg actgcctgta 480

cctcaacgtg tggacaccat acccccggcc tacatccccc acccctgtcc tcgtctggat 540

ctatgggggt ggcttctaca gtggggcctc ctccttggac gtgtacgatg gccgcttctt 600

ggtacaggcc gagaggactg tgctggtgtc catgaactac cgggtgggag cctttggctt 660

cctggccctg ccggggagcc gagaggcccc gggcaatgtg ggtctcctgg atcagaggct 720

ggccctgcag tgggtgcagg agaacgtggc agccttcggg ggtgacccga catcagtgac 780

gctgtttggg gagagcgcgg gagccgcctc ggtgggcatg cacctgctgt ccccgcccag 840

ccggggcctg ttccacaggg ccgtgctgca gagcggtgcc cccaatggac cctgggccac 900

ggtgggcatg ggagaggccc gtcgcagggc cacgcagctg gcccaccttg tgggctgtcc 960

tccaggcggc actggtggga atgacacaga gctggtagcc tgccttcgga cacgaccagc 1020

gcaggtcctg gtgaaccacg aatggcacgt gctgcctcaa gaaagcgtct tccggttctc 1080

cttcgtgcct gtggtagatg gagacttcct cagtgacacc ccagaggccc tcatcaacgc 1140

gggagacttc cacggcctgc aggtgctggt gggtgtggtg aaggatgagg gctcgtattt 1200

tctggtttac ggggccccag gcttcagcaa agacaacgag tctctcatca gccgggccga 1260

gttcctggcc ggggtgcggg tcggggttcc ccaggtaagt gacctggcag ccgaggctgt 1320

ggtcctgcat tacacagact ggctgcatcc cgaggacccg gcacgcctga gggaggccct 1380

gagcgatgtg gtgggcgacc acaatgtcgt gtgccccgtg gcccagctgg ctgggcgact 1440

ggctgcccag ggtgcccggg tctacgccta cgtctttgaa caccgtgctt ccacgctctc 1500

ctggcccctg tggatggggg tgccccacgg ctacgagatc gagttcatct ttggatcccc 1560

cctggacccc tctcgaaact acacggcaga ggagaaaatc ttcgcccagc gactgatgcg 1620

atactgggcc aactttgccc gcacagggga tcccaatgag ccccgagacc ccaaggcccc 1680

acaatggccc ccgtacacgg cgggggctca gcagtacgtt agtctggacc tgcggccgct 1740

ggaggtgcgg cgggggctgc gcgcccaggc ctgcgccttc tggaaccgct tcctccccaa 1800

attgctcagc gccaccgaca cgctcgacga ggcggagcgc cagtggaagg ccgagttcca 1860

ccgctggagc tcctacatgg tgcactggaa gaaccagttc gaccactaca gcaagcagga 1920

tcgctgctca gacctgtgac cccggcggga cccccatgtc ctccgctccg cccggccccc 1980

tagctgtata tactatttat ttcagggctg ggctataaca cagacgagcc ccagactctg 2040

cccatcccca ccccaccccg acgtcccccg gggctcccgg tcctctggca tgtcttcagg 2100

ctgagctcct ccccgcgtgc cttcgccctc tggctgcaaa taaactgtta caggcc 2156

1